(19) Europäisches Patentamt
European Patent Office
Office européen des brevets

(11) EP 1 707 201 A1

(12) # EUROPEAN PATENT APPLICATION

(43) Date of publication:
**04.10.2006 Bulletin 2006/40**

(21) Application number: **06112009.3**

(22) Date of filing: **30.03.2006**

(51) Int Cl.:
***A61K 31/421*** *(2006.01)* ***A61K 31/426*** *(2006.01)*
***A61P 17/00*** *(2006.01)* ***A61P 17/04*** *(2006.01)*
***A61P 17/06*** *(2006.01)* ***A61P 17/08*** *(2006.01)*

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **30.03.2005 DK 200500437**
**30.03.2005 DK 200500438**
**27.06.2005 DK 200500948**
**27.06.2005 DK 200500949**
**27.06.2005 US 694774 P**
**28.06.2005 US 695040 P**

(71) Applicant: **Astion Development A/S**
**2100 Copenhagen O (DK)**

(72) Inventor: **Weidner, Morten Sloth**
**2830, Virum (DK)**

(74) Representative: **Plougmann & Vingtoft A/S**
**Sundkrogsgade 9,**
**P.O. Box 831**
**2100 Copenhagen Ø (DK)**

(54) **Oxaprozin or a closely related compound for the treatment of eczema**

(57) The present invention relates to a novel principle of treating dermatological diseases comprising inhibiting several crucial steps in the inflammatory cascade including at least the inhibition of one or more of the enzymes Protein tyrosine kinase Syk, Protein tyrosine kinase ZAP-70 and phosphodiesterase IV (PDE-IV). The invention provides medicaments and methods for the treatment of inflammatory dermatological diseases, particularly eczemas, comprising Oxaprozin or a closely related compound or a salt thereof.

EP 1 707 201 A1

## Description

### FIELD OF THE INVENTION

The present invention relates to a pharmacological invention. The invention provides methods and medicaments for the treatment of a dermatological disease, particularly eczema, such as contact dermatitis, atopic dermatitis and hand eczema, by inhibiting multiple enzymes, particularly one or more of the enzymes, Protein tyrosine kinase Syk, Protein tyrosine kinase ZAP-70 and phosphodiesterase IV (PDE-IV).

### BACKGROUND OF THE INVENTION

Currently, effective and safe drugs for the treatment of contact dermatitis, atopic dermatitis and related eczemas are not available. Even the use of strong steroids does not effectively relieve pruritus associated with such diseases nor reduces the underlying inflammation during prolonged periods. To control itching, many dermatologists place patients on an antihistamine, which unfortunately also exerts sedative effects. Furthermore, in some types of atopic dermatitis, contact dermatitis and other eczemas, the pruritus is not histamine mediated and it does not respond well to histamine blockade. Since there is no effective treatment for most sufferers of contact dermatitis, atopic dermatitis and related eczemas, in particular with respect to treating pruritus, there is a large unmet need in those indications.

It has now been discovered that Oxaprozin, which hitherto has been recognised as a nonsteroidal anti-inflammatory drug (NSAID), effectively inhibits the enzymes protein tyrosine kinase Syk, protein tyrosine kinase ZAP-70 and phosphodiesterase IV (PDE-IV) in pharmacologically relevant doses and effectively reduces the symptoms and inflammation in an animal model of contact dermatitis.

It is generally acknowledged that the term "nonsteroidal anti-inflammatory drugs" is used to describe compounds with a molecular formula based on a substituted phenol or benzene ring and which pharmacologic actions principally are related to the inhibition of the enzyme cyclooxygenase-1 (Cox-1) and to some extent also to the inhibition of cyclooxygenase-2 (Cox-2) (*see* Greaves MW. Pharmacology and significance of nonsteroidal anti-inflammatory drugs in the treatment of skin diseases. J Am Acad Dermatol 1987 April;16(4):751-64). Whereas Cox-1 derived prostaglandins are not produced in skin, the Cox-2 enzyme produces prostaglandins at sites of inflammation for which reason the goal of pharmacologic anti-inflammatory therapy in the past has been to inhibit Cox-2 derived prostaglandins.

However, as discovered by the present inventor, Oxaprozin has a quite different pharmaco-dynamic and safe profile in comparison to other known NSAIDs.

The enzymatic activities of each of the enzymes; protein tyrosine kinase Syk, protein tyrosine kinase ZAP-70 and phosphodiesterase IV (PDE-IV) are crucial steps in the inflammatory process and operate at much higher hierarchic levels than the cyclooxygenase pathway. Therefore, Oxaprozin interferes with multiple crucial pathways in the inflammatory cascade, which renders this drug much more promising as an anti-inflammatory candidate, in particular with respect to the treatment of inflammatory dermatological diseases, such as eczemas, than previously expected.

Protein tyrosine kinases Syk and ZAP-70 are a class of enzymes that catalyze the transfer of a phosphate group from ATP to a tyrosine residue located on a protein substrate. They mediate the earliest detectable signalling response of the inflammation process upon activation of mast cells, T-cells and B-cells leading to multiple cascades of proinflammatory reactions. Protein tyrosine kinase Syk is involved in the cellular responses to degranulation, lipid mediator synthesis and cytokine production in inflammatory cells, and it is expected that protein tyrosine kinase Syk takes part in allergic or inflammatory reactions through controlling the functions of mast cell, basophils, and eosinophils. The protein tyrosine kinase ZAP-70 is required for T-cell development and T-cell antigen receptor function.

The PDE-IV enzymes belong to the family of phosphodiesterases (PDE's) which are responsible for the hydrolysis of intracellular cyclic adenosine and guanosine monophosphate (cAMP and cGMP, respectively). The type IV phosphodiesterase is a cAMP-specific enzyme localized in airway-smooth muscle cells as well as in immune and inflammatory cells. The type IV enzyme is a key enzyme in the hydrolysis of cAMP in mast cells, basophils, eosinophils, monocytes and lymphocytes.

Furthermore, in addition to the enzyme systems recognised by the present inventor, Oxaprozin inhibits both anandamide hydrolase (a fatty acid amide hydrolase) in neurons and NF-kappaB activation in inflammatory cells. Oxaprozin also induces apoptosis of activated monocytes in a dose-dependent manner *(*Dallegri, Franco. A review of the emerging profile of the anti-inflammatory drug oxaprozin. Expert Opin Pharmacother 2005 May; 6(5): 777-85*).*

Obviously, the present inventor has recognised the very great pharmacological potential of Oxaprozin, at least with respect to treating dermatological diseases, in that this single compound is able to modify several of the crucial and principal pathways of the immunological response *in vivo,* which usually requires the administration of several compounds to obtain the same effect.

While Oxaprozin and NSAIDs have been used for a long time in the treatment of systemic inflammatory diseases, like osteoarthritis and rheumatoid arthritis, the utility of Oxaprozin in the treatment of inflammatory dermatological diseases, such as eczemas, has not been emphasized or demonstrated before.

Some NSAIDs have been suggested and developed for the treatment of specific dermatological diseases. To be mentioned is acetylsalicylic acid, indomethacin and parfenac (marketed as bufexamac®) that have been used in the treatment of dermatological diseases for a long time. Acetylsalicylic acid has been used in the treatment of pruritus in atopic eczema; indomethacin has been used in the treatment of sunburned skin; and parfenac has been used in the treatment of eczema, dermatitis and perianal pruritus *(*Greaves MW. Pharmacology and significance of nonsteroidal anti-inflammatory drugs in the treatment of skin diseases, J Am Acad Dermatol 1987 April;16(4):751-64*).*

Salicylic acid has been used in the treatment of psoriasis *(*Going SM, Guyer BM, Jarvie DR, Hunter JA. Salicylic acid gel for scalp psoriasis. Clin Exp Dermatol 1986 May;11 (3):260-2*)* and in the treatment of pruritus *(*Thomsen JS, Simonsen L, Benfeldt E, Jensen SB, Serup J. The effect of topically applied salicylic compounds on serotonin-induced scratching behaviour in hairless rats. Exp Dermatol 2002 August;11 (4):370-5*).*

Topically applied indomethacin has been shown to respond in an animal model of contact dermatitis *(*Lowe NJ, Virgadamo F, Stoughton RB. Anti-inflammatory properties of a prostaglandin antagonist, a corticosteroid and indomethacin in experimental contact dermatitis. BrJ Dermatol 1977 April; 96(4):433-8*)* and topical treatment with flubiprofen reduces human skin inflammation *(*Black AK, Hensby CN, Greaves MW. The effect of topical flurbiprofen on human skin inflammation [proceedings]. Br J Clin Pharmacol 1980 January; 9(1):125P*).*

However, some NSAIDS have failed to show any clear ability to treat the inflammation of certain skin diseases. For example, one study demonstrates that topically applied indomethacin has poor effect in relieving the erythema and oedema in moderate to severe inflammation following treatment with cryotherapy. In comparison, the topical application of the steroid, clobetasol propionate, proved to have effect *(*Humphreys F, Spiro J. The effects of topical indomethacin and clobetasol propionate on post-cryotherapy inflammation. BrJ Dermatol 1995 May;132(5):762-5*).* Green and Shuster demonstrate that topical 1% indomethacin had no effect on chronic stable plaque psoriasis in human studies *(*Green CA, Shuster S. Lack of effect of topical indomethacin on psoriasis. BrJ Clin Pharmacol 1987 September;24(3):381-4*).*

Unfortunately, the topical use of various NSAIDs is associated with significant cutaneous side effects. For example, Bufexamac is marketed for the treatment of pruritus and contact allergy, but the compound itself is reported to cause contact allergy. Furthermore, it is reported that aspirin and indometacin may induce urticarial reactions, whereas piroxicam can lead to phototoxic or photoallergic dermatitis. Ketoprofen and Bufexamac are recognised as major contact allergens *(*Gebhardt M and Wollina U. Cutaneous side-effects of nonsteroidal anti-inflammatory drugs (NSAID) in Z Rheumatol 1995 November;54(6):405-12*).* Diclofenac is another NSAID that is associated with cutaneous side-effects when applied topically in that irritant-type contact dermatitis may develop *(*Rivers JK and McLean DI. An open study to assess the efficacy and safety of topical 3% diclofenac in a 2.5% hyaluronic acid gel for the treatment of actinic keratoses. Arch Dermatol 1997 October;133(10):1239-42*).*

Thus, the findings of the present inventor are quite surprising because unlike other used NSAIDs, Oxaprozin is found very effective and safe in treating dermatological diseases. In considering the present invention and the treatment of inflamed tissue of the skin, Oxaprozin should no longer be regarded as a typical NSAID, because its principal pharmacodynamic properties relevant to inflamed skin do not include cyclooxygenase (Cox-1/Cox-2) inhibition. In fact, Oxaprozin is one of the NSAIDs with poorest Cox-2 inhibitory activity and selectivity for Cox-2 inhibition *(*Kawai S. Cyclooxygenase selectivity and the risk of gastrointestinal complications of various non-steroidal anti-inflammatory drugs. A clinical consideration. In Inflamma. Res. Supplement 2 (1998) S102-S106*).* Thus, the present inventor believes that Oxaprozin does not belong to the group of Cox-2 inhibitors.

The patent applications, US2005014729 and WO05009342 (both of Pharmacia Corporation) relate to methods for treating dermatological diseases, preferably acne, by administering a Cox-2 inhibitor. The technical teaching found herein concerns the treatment of skin diseases by administering a Cox-2 inhibitor. It is further taught that any compound having Cox-2 inhibition can be used and that such compounds preferably are selective Cox-2 inhibitors, but that NSAIDs can also be applied. An exhaustive list of NSAIDs is mentioned in US2005014729 and WO05009342 and includes Oxaprozin. However, the disclosures in US2005014729 and WO05009342 are unclear and ambiguous in nature and the applications fail to teach the specific utility of Oxaprozin for the treatment of skin diseases. Moreover, the invention as defined in US2005014729 and WO05009342 is very broadly defined and is more conceptual than substantial, in that all substances with Cox-2 inhibition are claimed to be effective in the treatment of all skin diseases despite the fact that some of them are already marketed for the treatment of a dermatological disease and others have failed to show effect in the treatment of dermatological diseases.

The following patent applications also relate to the treatment of dermatological diseases by administering various NSAIDs, but they all fail to directly and unambiguously disclose the utility of Oxaprozin for that purpose.

The patent application, JP7316075A2 (POLA CHEM IND INC) discloses dermatological formulations containing an antiphlogistic sedative drug and 10-20% of a water-retaining agent. The formulations are intended for the treatment of dry skin or for the treatment of dry skin associated with a skin disease, such as atopic dermatitis. Various drugs are suggested to act as the antiphlogistic sedative drug, namely NSAIDs including Oxaprozin, but particularly parfenac (bufexamac), indomethacin, ibuprofen and tenoxicam; steroids, such as dexamethasone, clobetasone, prednisolone

and hydrocortisone; and vitamin A derivatives, such as tocoretinate. Thus, many antiphlogistic sedative drugs are suggested despite that their pharmacological properties are very different and unrelated in nature.

US2005232957A1 of Katz K (published 20-10-2005) relates to pharmaceutical compositions for the treatment of dry skin, such as in a subject suffering from atopic dermatitis and contact dermatitis. The compositions comprise specific (such as rofecoxib) or non-specific Cox inhibitors (an exhaustive list of Cox inhibitors including Oxaprozin is mentioned). The pharmacological principle of the invention is based on the expectation that Cox inhibitors enhances the concentration of sodium chloride in sweat and/or the water-binding capacity of keratins in the epidermis and thereby moistures the skin.

WO9735573A2 (THE BOOTS COMPANY PLC) relates to the treatment of pruritus by administering one or more NSAIDs selected from bendazac, benzydamine, diclofenac, fenbufen, indomethacin, ketoprofen, naproxen, piroxicam and sulindac.

WO9511017A1 (THE BOOTS COMPANY PLC) relates to the treatment of pruritus by administering ibuprofen or flurbiprofen.

WO02074290 (AGIS INDUSTRIES (1983) LTD) relates to a method of treating the skin disease, rosacea, by topical administration of an NSAID.

Thus, the present invention provides an effective and safe treatment of eczemas. This is quite surprising because many attempts have been made over time to apply NSAIDs in the treatment of dermatological diseases, but the success has been limited so far because of too low effect and significant skin irritation.

## SUMMARY OF THE INVENTION

Surprisingly, the present inventor has found that Oxaprozin, unlike other NSAIDs possess a strong therapeutic potential in the management of dermatological diseases, where the inhibition of at least one of the enzymes Protein tyrosine kinase Syk; Protein tyrosine kinase ZAP-70; and phosphodiesterase IV (PDE-IV) in the skin will lead to alleviation, suppression or removal of the inflammation of a dermatological disease. Such dermatological diseases are in particular thought to include various forms of eczemas, such as contact dermatitis, atopic dermatitis and hand eczema. Furthermore, such inhibition will also remove, alleviate or reduce the predominant symptom of these diseases, namely pruritus, and other symptoms such as erythema and oedema.

Experimental data shown herein clearly verify the significant immediate and complete alleviation of the characteristic symptoms of inflammation associated with various forms of eczemas (See examples 2 (irritant contact dermatitis and atopic dermatitis), 3 (insect bite inflammation), 4 (psoriasis) and 5 (oxazolone induced contact dermatitis in mice). The human data shown herein clearly demonstrates the significant immediate and complete alleviation of pruritus in patients suffering from contact dermatitis, atopic dermatitis and psoriasis. Furthermore, erythema and scaling of the skin were also improved during the first 1-2 weeks of treatment indicating a therapeutic effect on the underlying disease causing the pruritus. Oxaprozin displayed, at clinically relevant doses, a strong inhibiting effect at least comparable to that achieved with the strong steroid betamethasone-17-valerate.

The present inventor puts forward the hypothesis that the convincing effect observed with Oxaprozin is associated with the excellent pharmacological properties of Oxaprozin, namely the inhibition, in micromolar concentrations of one or more of the enzymes; Protein tyrosine kinases Syk, Protein tyrosine kinases ZAP-70 and PDE-IV enzyme. Such micromolar concentrations of Oxaprozin are expected to be present in skin cells following topical administration, but pharmacologically active doses are also expected to be present following systemic administration.

Contrary to existing therapeutic agents used for treating inflammatory dermatological diseases, such as eczemas, the treatments according to the present invention have the advantage of not being likely to be associated with any serious side effects, as Oxaprozin has been shown to be safe and well tolerated by the organism in pharmacologically relevant doses. For example, Oxaprozin (in the form of its monoethanolamine salt) does not produce any cutaneous side-effects in the form of sensitization, phototoxic reaction, or acute dermal irritation.

Accordingly, a first aspect of the invention relates to the use of Oxaprozin or a closely related compound or a salt thereof for the preparation of a medicament formulated for topical administration to skin or for systemic administration for the treatment of an inflammatory dermatological disease of a subject, such as for the treatment of the inflammation of a dermatological disease, in particular for the treatment of eczemas.

According to the present invention, the inflammatory dermatological diseases to be treated encompass those where at least one of the enzymes protein tyrosine kinase Syk, protein tyrosine kinase ZAP-70 and PDE-IV play a role in mediating the inflammatory dermatological disease. It is generally expected that such inflammatory dermatological diseases are caused by an initial hypersensitivity reaction in the skin, such as type-I allergy or type-IV allergy reactions in skin.

Therefore, a still further aspect of the invention relates to the use of Oxaprozin or a closely related compound or a salt thereof for the preparation of a medicament formulated for topical administration to skin or for systemic administration for the inhibition of one or more of the enzymes selected from Protein tyrosine kinase Syk; Protein tyrosine

kinase ZAP-70 and/or PDE-IV in a subject diagnosed with or having an inflammatory dermatological disease.

According to the underlying pharmacological principle of the invention, the systemic administration or topical administration of Oxaprozin or a closely related compound or a salt thereof effectively alleviate, remove or prevent specific symptoms caused by or associated with the inflammation or the hypersensitivity reaction in a subject diagnosed or suffering from an inflammatory dermatological disease. For example, pruritus is a predominant and highly unpleasant symptom of many inflammatory diseases.

Therefore, an important aspect of the invention relates to the treatment or prevention of pruritus and optionally other significant symptoms associated with an inflammatory dermatological disease, such as particularly oedema, erythema and scaling. Accordingly, the invention provides the use of Oxaprozin or a closely related compound or a salt thereof for the preparation of a medicament formulated for systemic administration or for topical administration to skin for the alleviation, removal or prevention of one or more of the symptoms in skin selected from pruritus; erythema; oedema; and/or scaling in a subject having an inflammatory dermatological disease, such as particularly a subject having eczema.

The technical effect achieved by Oxaprozin can also be achieved by administering drug agents, which, like Oxaprozin, inhibit at least two of the above-mentioned enzymes or all three of the above-mentioned enzymes. Such drug agents are at least thought to encompass compounds with a molecular structure closely related to Oxaprozin. Additionally, other drug agents sharing the same pharmacodynamic profile, either as a single drug agent or in combination of two or three drug agents, can provide the same effect as Oxaprozin with respect to the treatment of dermatological diseases.

Therefore, still another aspect of the invention relates to uses, where at least two or three of the enzymes protein tyrosine kinase Syk, protein tyrosine kinase ZAP-70 and PDE-IV play a role in mediating the disease. Therefore, the invention also relates to the use of at least two inhibitors selected from the group consisting of an inhibitor of Protein tyrosine kinase Syk; Protein tyrosine kinase ZAP-70; and PDE-IV enzyme for the preparation of a medicament for the treatment of an inflammatory dermatological disease in a subject. The inhibition may be accomplished by one single drug agent or by a combination of two or three drug agents acting in combination.

In the various aspects of the invention, the treatment or prevention of an inflammatory dermatological disease encompasses:

1) the treatment of eczemas;

2) the treatment of hypersensitivity reactions in skin;

3) the treatment of type-IV allergies in skin;

4) the treatment of type-I allergies in skin;

5) the treatment or prevention of contact dermatitis;

6) the treatment or prevention of allergic contact dermatitis;

7) the treatment or prevention of irritant contact dermatitis;

8) the treatment of atopic dermatitis;

9) the treatment of hand eczema;

10) the alleviation, removal or prevention of one or more of the following symptoms of the skin selected from the group consisting of pruritus; oedema; erythema; and scaling in a subject with inflammatory dermatological disease.

## DETAILED DESCRIPTION OF THE INVENTION

There is provided a safe and effective treatment of inflammatory dermatological diseases, particularly eczemas, by selecting drug agents or optionally combinations of drug agents, which inhibit several important enzyme systems involved in the inflammatory cascade in skin cells. One specific drug agent has been found to possess such advantageous properties. Therefore, in a first aspect the invention provides the use of Oxaprozin or a closely related compound or a pharmaceutically acceptable salt thereof for the preparation of a medicament formulated for topical administration to skin for the treatment of an inflammatory dermatological disease in a subject, wherein the closely related compound is defined as presented below. According to an alternative aspect, Oxaprozin or the closely related compound or a pharmaceutically acceptable salt thereof may be systemically administered to the subject.

In view of the pharmacological principle of the invention, another aspect relates to the use of Oxaprozin or a closely related compound or a pharmaceutically acceptable salt thereof for the preparation of a medicament formulated for topical administration to skin or systemic administration for the inhibition of at least two of the enzymes selected from the group consisting of an inhibitor of Protein tyrosine kinase Syk; Protein tyrosine kinase ZAP-70; and PDE-IV enzyme in a subject with an inflammatory dermatological disease. It should be understood that the inhibition of said enzymes take place in skin cells.

According to a broader aspect of the invention, the effect of Oxaprozin can be achieved by administering a drug agent with similar pharmacodynamic properties to Oxaprozin or a combination of two or three drug agents that in common has similar pharmacodynamic properties to Oxaprozin. Therefore, the invention also relates to the use of at least two inhibitors selected from the group consisting of an inhibitor of protein tyrosine kinase Syk; protein tyrosine kinase ZAP-70; and PDE-IV enzyme for the preparation of a medicament for the treatment of an inflammatory dermatological disease in a subject.

That is to say that the following inhibitors can achieve the desired effect:

1) an inhibitor of Protein tyrosine kinase Syk and Protein tyrosine kinase ZAP-70;
2) an inhibitor of Protein tyrosine kinase Syk and PDE-IV enzyme;
3) an inhibitor of Protein tyrosine kinase ZAP-70 and PDE-IV enzyme; or
4) an inhibitor of Protein tyrosine kinase Syk and Protein tyrosine kinase ZAP-70 and PDE-IV enzyme, where one single drug agent or a combination of two or three different drug agents may act as the above-mentioned inhibitors.

The following definitions are used herein:

The phrase "closely related compound" is meant to define compounds resembling Oxaprozin in its molecular structure and which are further defined below.

The term "an inhibitor of Protein tyrosine kinase Syk" refers to any agent that inhibits the catalytic activity of Protein tyrosine kinase Syk *in vitro.* Such in vitro assays are described *in* Shichijo M, Yamamoto N, Tsujishita H, Kimata M, Nagai H, Kokubo T. Inhibition of syk activity and degranulation of human mast cells by flavonoids. Biol Pharm Bull 2003 December;26(12):1685-90 *and in* Zhang J, Berenstein E, Siraganian RP. Phosphorylation of Tyr342 in the linker region of Syk is critical for Fc epsilon RI signalling in mast cells. Mol Cell Biol 2002 December;22(23):8144-54. The inhibitory activity of Protein Tyrosine Kinase SYK may be determined at MDS Pharma Service using test method number 155761.

Examples on Syk inhibitors include, without limitation, NVP- QAB205 (Novartis); purine-2-benzamine derivatives such as those described in U.S. Patent No. 6,589, 950; pyrimidine-5-carboxamide derivatives such as those described in PCT Publication No. WO 99/31073; 1,6-naphthyridine derivatives such as those described in U.S. Patent Publication No. 2003/0229090; BAY 61-3606 (Bayer); piceatannol; 3,4- dimethyl-10-(3 aminopropyl)-9-acridone oxalate); and combinations thereof.

The term "an inhibitor of Protein tyrosine kinase ZAP-70" refers to any agent that inhibits the catalytic activity of the Protein tyrosine kinase ZAP-70 *in vitro* as may be determined using a method described in Huckle WR, Dy RC, Earp HS. Calcium-dependent increase in tyrosine kinase activity stimulated by angiotensin II. Proc Natl Acad Sci U S A 1992 September 15;89(18):8837-41 *and in* Isakov N, Wange RL, Watts JD, Aebersold R, Samelson LE. Purification and characterization of human ZAP-70 protein-tyrosine kinase from a baculovirus expression system. J Biol Chem 1996 June 28;271 (26):15753-61. The inhibitory activity of Protein Tyrosine Kinase ZAP may be determined at MDS Pharma Service using test method number 155987.

The term "an inhibitor of PDE-IV enzyme" refers to any agent that inhibits the catalytic activity of the PDE-IV enzyme *in vitro* as determined by a test method described in Nicholson CD, Challiss RA, and Shahid M. Differential modulation of tissue function and therapeutic potential of selective inhibitors of cyclic nucleotide phosphodiesterase isoenzymes. Trends Pharmacol Sci 1991 January;12(1):19-27 *and in* Cortijo J, Bou J, Beleta J, Cardelus I, Llenas J, Morcillo E et al. Investigation into the role of phosphodiesterase IV in bronchorelaxation, including studies with human bronchus. Br J Pharmacol 1993 February;108(2):562-8. The inhibitory activity of Phosphodiesterase PDE IV may be determined at MDS Pharma Service using test method number 154000.

Examples of PDE-IV inhibitors that are known under their INN names are; Anagrelide; Amrinone (also named inamrinone); Arofylline; Atizoram; Benafentrine; Cilomast; Cilostazol; Cipamfylline; Denbufylline; Dipamfylline; Enprofylline; Filaminast; hydroxypumafentrine (Altana); Indolidan; Ibudilast; Lixazinone; Lirimilast; Mesopram; Mopidamol; Motapizone; Nitraquazone; Olprinone; Oxagrelate; Piclamilast; Pimobendan; Propentophylline; Pumafentrine; Tibenelast ; Tolafentrine; Trequinsine; Roflumilast; and Rolipram.

According to the present invention, the inhibitors are meant to encompass small organic molecules, such as typically with a molecular weight up to 800 Dalton.

The term "drug agent" is meant to define small organic molecules, typically having a molecular weight up to

800 Dalton, that has a pharmacologically activity, particularly acting as an inhibitor of one or more of the protein kinases Syk and Za-70 and the PDE-IV enzyme.

The terms "inhibition" and "inhibits" are interchangeable terms that are meant to define that the enzyme activity *in vitro* of the Protein tyrosine kinase Syk; Protein tyrosine kinase ZAP-70 and/or the PDE-IV enzyme is reduced by at least 30%, such as at least 40% by adding Oxaprozin or another inhibitor in a molar concentration of no more than 500 μM. Preferably, the concentration of Oxaprozin or another inhibitor that provides 50% inhibition (IC50%) is not greater than 500 μM, preferably not greater than 300 μM, such as not greater than 250 μM, 200μM, 150μM, 100 μM or 50μM.

In the context of the present invention, the term "an inflammatory dermatological disease" is meant to define a dermatological disease, where at least one of the enzymes selected from the group consisting of Protein tyrosine kinase Syk; Protein tyrosine kinase ZAP-70 and PDE-IV enzyme plays a role in mediating the dermatological disease.

The phrase "where at least one of the enzymes selected from the group consisting of Protein tyrosine kinase Syk; Protein tyrosine kinase ZAP-70 and PDE-IV enzyme play a role in mediating the dermatological disease" is meant to define various dermatological diseases where over-expression or excessive amounts of these enzymes play a role. The following dermatological diseases are meant as non-limiting examples of such dermatological diseases: acne vulgaris, adult eczema, alopecia, allergic contact dermatitis, allergic dermatitis, allergic contact eczema, asteatotic eczema, atopic eczema, hand eczema, atopic dermatitis, childhood eczema, chronic dermatitis of hands or feet, contact dermatitis, contact eczema, discoid eczema, drug-induced skin reactions, dermatitis herpetiformis, discoid lupus erythematosus, epidermolysis bullosa, erythroderma, erythema nodosum, erythema multiforme, hand eczema, hand and foot dermatitis, ichthyosis vulgaris, infantile eczema, insect bite inflammation, keratoconus, keratosis pilaris, lichen simplex chronicus, lichen planus, nummular dermatitis, melanomas, over-treatment dermatitis, pemphigus, pemphigoid, photodermatoses, pityriasis rosea, pyoderma gangrenosum, pompholyx, psoriasis, prurigo nodularis, rosacea, scabies, seborrheic dermatitis, seborrhea, scleroderma, Sjogren's Disease, stasis dermatitis, subacute cutaneous lupus erythematosus, sunburn, systemic lupus erythematosus, vitiligo and urticaria.

The phrase "treating the inflammation of a dermatological disease" is meant to define that the treatment as described herein reduces, removes of prevents the inflammation associated with a dermatological disease. Characteristic signs of inflammation in a dermatological disease are oedema, erythema and scaling, which this invention reduces, removes or prevents.

The phrase "formulated for topical administration to skin" and topical administration is meant to define interchangeable terms that encompasses the formulation of the active ingredient of this invention (Oxaprozin or a closely related compound) into a dosage form that can be applied to skin of a subject and which result in the local presence of the active ingredient in the skin. The phrase "local presence of the active ingredient in skin" is meant to include topical administration of the active ingredient to skin with the presumption that systemic uptake of the active ingredient is limited or nil. Thus, it is intended that less than 25% by weight, such as less than 20% by weight, such as less than 15% by weight, such as less than 10%, 8%, 5% and 3% by weight, of the topically administered active ingredient enters the blood stream or is recovered in urine and faeces. Dosage forms for topical application to skin typically encompass emulsions (creams), ointments, gels, liniments, powders and solutions.

The phrase "formulated for systemic administration" is meant to define the formulation of the active ingredient of this invention (Oxaprozin or a closely related compound) into a dosage form, which when administered to a subject results in the systemic uptake of the active ingredient into the blood. The phrase "systemic uptake of the active ingredient" or "systemic administration" is interchangeable terms and is meant to include any form of administration of the active ingredient resulting in the entrance of the active ingredient into the blood stream. Therefore, the active ingredient may be administered by the per-oral, transdermal, transmucosal or the parenteral route, preferably by the oral route.

The term "skin cells" is meant to encompass cells present in stratum corneum, dermis and epidermis. When considering inflammatory dermatological diseases, such cells may include cells that constitute the inflammation in skin, such as T-cells, macrophages, mast cells, Langerhans cells and neutrophils.

The term "skin" is meant to include skin of the entire body including the scalp, the forehead, the head, arms, legs, breast and so forth. The term "skin" is also meant to include various layers of the skin, such as stratum corneum, epidermis and dermis.

The term "subject" for purposes of treatment includes any subject, but preferably a subject who is in need of the treatment of an inflammatory dermatological disease. For purposes of prevention, the subject is any subject, and preferably a subject that is at risk of, or is predisposed to, developing an inflammatory dermatological disease. The subject is typically an animal, and yet more typically is a mammal. "Mammal", as used herein, refers to any animal classified as a mammal, including humans, domestic and farm animals, zoo, sports, or pet animals, such as dogs, horses, cats, cattle, etc. Preferably, the mammal is a human. Typically, a subject is a human diagnosed or suffering from various forms of eczemas, such as contact dermatitis, atopic dermatitis and hand eczemas. In preferred embodiments the subject is a human, a dog, a cat or a horse.

The terms "treat", "treating" and "treatment" as used herein are meant to include alleviating or abrogating an inflammatory dermatological disease or its attendant symptoms and alleviating or eradicating the cause of the disease

itself.

The terms "prevent", "preventing" and 'prevention", as used herein, refer to a method of delaying or precluding the onset of symptoms of an inflammatory dermatological disease, such as preventing the reoccurrence of inflammation or pruritus.

The term "therapeutically effective amount" refers to the amount of Oxaprozin or a related compound that will elicit the biological or medical response of a tissue, system, animal or human that is being sought by the researcher, veterinarian, medical doctor or other clinician. The term "therapeutically effective amount" includes that amount of a compound that, when administered, is sufficient to prevent the development of, or to some extent alleviation of, pruritus being treated. The therapeutically effective amount will vary depending on the compound, the disease and its severity and the age, weight, etc., of the mammal to be treated. With respect to topical administration of an effective amount of Oxaprozin or a closely related compound or a salt thereof to skin, it is considered to apply a dermatological formulation comprising between 0.1% to 10% by weight of Oxaprozin or a closely related compound or a salt thereof to the affected skin areas for 1 to 4 times daily. In methods or uses of the invention where Oxaprozin or a derivative thereof is administered systemically, a daily dose level of 1-200 mg/(kg body weight) is applied depending on the duration of the treatment, the condition to be treated, the formulation and the bioavailability. In a preferred embodiment of the invention the daily dose level is 5-100 mg/(kg body weight). In an even more preferred embodiment of the invention the daily dose level is 10-50 mg/(kg body weight).

In presently interesting embodiments of the invention, the treatment or prevention of an inflammatory dermatological disease comprises inhibition of one or more of the enzymes Protein tyrosine kinase Syk; Protein tyrosine kinase ZAP-70 and/or PDE-IV in the subject, such as in the skin cells of said subject. As recognised by the present inventor, the treatment of an inflammatory dermatological disease is improved by inhibiting multiple targets rather than one target, for which reason an improved embodiment of the invention preferably comprises inhibition of at least two of the above-mentioned enzymes, such as the Protein tyrosine kinase Syk and the Protein tyrosine kinase ZAP-70, the Protein tyrosine kinase Syk and the PDE-IV enzyme; the Protein tyrosine kinase ZAP-70 and the PDE-IV enzyme.

In order to provide a general treatment principle for dermatological diseases, such as eczemas, it may be advantageous to inhibit all of the above-mentioned three enzymes.

In currently interesting embodiments of the invention, the treatment of an inflammatory dermatological disease encompasses those diseases where hypersensitivity reactions are part of the pathology, such as particularly type-IV or type-I allergy reactions. Currently interesting examples of such dermatological diseases are eczemas including dermatitis, such as contact dermatitis, atopic dermatitis and hand eczema.

The meaning of the word "eczema" is "flowing over" which describes some of the above inflammatory changes. The word eczema is meant to include dermatitis and the two terms are used somewhat interchangeable because in both conditions similar inflammatory conditions occur in the skin.

Eczema generally refers to several conditions that share a pattern of changes in the surface of the skin. Eczema appears as an episode of skin inflammation that may present itching, scaling and erythema of the skin. When it develops into a long-term condition (chronic eczema), it leads to thickening skin, scaling, flaking, dryness and colour changes. There are many types of eczemas, depending on the cause, shape and location of the rash. Most eczemas are related to allergies or to contact with irritating chemicals. The term "dermatitis" is meant to define inflammation that affects the epidermis and the superficial dermis and is characterised by featuring red and hot skin. Sometimes, the dermatitis further features oedema producing intradermal vesicles; weeping of fluid on to the surface, crusting, scaling and fissuring.

Eczemas are divided into endogenous eczemas and exogenous eczemas. Endogenous eczemas encompass at least atopic dermatitis including various forms thereof; infantile eczema, childhood eczema, adult eczema, keratosis pilaris, ichthyosis vulgaris, pompholyx, discoid eczema and nummular eczema. Exogenous eczemas encompass at least allergic contact dermatitis, irritant contact dermatitis and hand eczema.

In current embodiments of the invention, the invention encompasses the treatment or prevention of inflammatory dermatological diseases that are associated with or caused by type-IV allergies in skin, such as typically contact dermatitis. Contact dermatitis is an inflammation of the skin caused by direct contact with an irritating or allergy-causing substance (irritant or allergen) in the same individual over time and is meant to include irritant contact dermatitis, allergic contact dermatitis and over-treatment dermatitis.

Irritant contact dermatitis is the most common type of contact dermatitis and involves inflammation resulting from contact with acids, alkaline materials such as soaps and detergents, solvents, or other chemicals. The reaction usually resembles a burn.

Allergic contact dermatitis is caused by exposure to a material to which the person has become hypersensitive or allergic, such as to nickel, plants, medications, rubber, laundry detergents, rhus oils, cosmetics, fabrics and clothing, solvents, adhesives, fragrances, perfumes and chemicals. The skin inflammation in contact dermatitis varies from mild irritation and erythema to open sores, depending on the type of irritant, the body part affected, and the sensitivity of the individual.

Over-treatment dermatitis is a form of contact dermatitis that occurs when treatment for another skin disease

causes irritation.

Skin suffering from contact dermatitis exhibits the following characteristic symptoms; Itching (pruritus) of the skin in exposed areas; skin erythema or inflammation in the exposed area; tenderness of the skin in the exposed area; localized swelling of the skin; warmth of the exposed area (may occur); skin lesion or rash at the site of exposure; lesions of any type: erythema, rash, papules (pimple-like), vesicles, and bullae (blisters).

Thus, the medical phrase "treatment or prevention of contact dermatitis" refers to the treatment or prevention of characteristic symptoms of contact dermatitis, such as pruritus, skin erythema; skin inflammation, localised swelling of the skin, skin rash, papules, vesicles and blisters and to the treatment or prevention of various types of contact dermatitis, such as irritant dermatitis, allergic contact dermatitis and over-treatment dermatitis.

In another interesting embodiment, uses and methods encompass the treatment or prevention of atopic dermatitis (i.e., eczema) that is a chronic pruritic skin condition usually beginning in infancy. The predominant symptom is intense pruritus. Atopic dermatitis is also an allergic skin disease of dogs which is caused by immunological hypersensitivity to common substances in the environment such as house dust mites. Atopic dermatitis may occur as infantile eczema, childhood eczema and adult eczema.

The skin in atopic dermatitis may show various characteristic signs according to the stage of disease. Acute disease is characterised by erosions with serous exudate or intensely itchy papules and vesicles on an erythematous base. The subacute stage is characterized by scaling, excoriated papules, or plaques over erythematous skin. The chronic phase is characterised by the presence of lichenification and pigmentary changes (increased or decreased) with excoriated papules and nodules. Lesions may become secondarily infected because of scratching.

Another manifestation of atopic dermatitis is Ichthyosis vulgaris, which is observed in one third or more of patients with atopic dermatitis. Characterizing features are hyperlinear palms and soles and polygonal fishlike scales, especially on the lower legs.

Still another manifestation is keratosis pilaris that is characterized by asymptomatic horny follicular papules on the extensor surfaces of the upper arms, buttocks, and anterior thighs.

Still another manifestation of atopic dermatitis is hand and foot dermatitis, which may be the only manifestation in adults and adolescents. Fissuring of the palms, soles, and fingers often occurs.

Still another manifestation of atopic dermatitis is keratoconus, which is observed in severe cases. A cone-shaped cornea (requiring corneal transplant) may develop in the second or third decade a subject's of life.

Atopic dermatitis may be associated with the following features; facial erythema, perioral pallor, infraorbital fold (i.e., Dennie-Morgan line), dry skin, increased palmar linear markings, pityriasis alba (hypopigmented asymptomatic areas on face and shoulders) and pilaris.

Thus, the medical phrase "treatment or prevention of atopic dermatitis" is meant to define the treatment or prevention of characteristic symptoms of the various stages of atopic dermatitis as mentioned above, such as pruritus, itchy papules on an erythematous skin, scaling erythematous skin, lichenification, pigmentary changes with papules and nodules and to the treatment of associated manifestations, such as Ichthyosis vulgaris, keratosis pilaris, hand and foot dermatitis and keratoconus.

Thus, the treatment or prevention of atopic dermatitis is meant to encompass the treatment or prevention of infantile eczema, childhood eczema, adult eczema, ichthyosis vulgaris, keratosis pilaris, hand and foot dermatitis and keratoconus.

Still another interesting embodiment of the invention encompasses the treatment or prevention of hand eczema. Limited to the hands, this type of eczema can be related to atopic eczema or it can occur because of repeated hand washing or exposure to strong detergents. Thus, hand eczema can also be related to irritant contact dermatitis. Occasionally, hand eczema is caused by an allergy, such as allergy to latex.

Still another interesting embodiment relates to the treatment of nummular eczema that refer to eczema presenting coin sized patches of irritated skin, typically on the legs, arms or chest. It usually occurs in adults. It can be related to atopic dermatitis and, less often, allergic contact dermatitis. In a few cases, it represents an allergic reaction to a fungal infection such as athlete's foot. In this case, nummular eczema still appears typically on arms, legs or chest, even if the fungal infection is elsewhere on the body.

Still other embodiments refer to the treatment or prevention of eczemas, which do not typically involve a hypersensitivity reaction, such as asteatotic eczema, stasis dermatitis, lichen simplex chronicus, prurigo nodularis, psoriasis, seborrheic dermatitis and seborrhea.

Thus, in presently interesting embodiments of the invention, the dermatological disease refers to various types of eczemas which at least encompass the following conditions: atopic dermatitis, hand eczema, infantile eczema, childhood eczema, adult eczema, keratosis pilaris, ichthyosis vulgaris, hand and foot dermatitis, keratoconus, pompholyx, discoid eczema, nummular eczema, allergic contact dermatitis, irritant contact dermatitis, over-treatment dermatitis, asteatotic eczema, stasis dermatitis, lichen simplex chronicus, prurigo nodularis, seborrheic dermatitis, seborrhea and psoriasis.

In more preferred embodiments of the invention, the dermatological disease refers to atopic dermatitis, hand

eczema, infantile eczema, childhood eczema, adult eczema, keratosis pilaris, ichthyosis vulgaris, hand and foot dermatitis, keratoconus, pompholyx, discoid eczema, nummular eczema, allergic contact dermatitis, irritant contact dermatitis, over-treatment dermatitis and hand eczema.

According to the underlying pharmacological principle of this invention, methods and uses of the invention comprises inhibition of one or more of the enzymes selected from the group consisting of Protein tyrosine kinase Syk; Protein tyrosine kinase ZAP-70 and PDE-IV enzyme in inflamed skin cells of a subject diagnosed with, or suffering from or in the risk at developing an inflammatory dermatological disease.

A predominant symptom of the above-mentioned eczemas is pruritus (itching of skin) and the present inventor has demonstrated complete relief of this symptom in subjects suffering from various types of eczemas mentioned herein, particularly contact dermatitis and atopic dermatitis. Furthermore, it was found that further symptoms were relieved, which means that uses and methods herein relates to alleviation, removal or prevention of one or more symptoms selected from the group consisting of pruritus, erythema, scaling and oedema in a subject having an inflammatory dermatological disease, such as eczema.

Therefore, a further aspect of the invention relates to the treatment or prevention of one or more symptoms selected from the group consisting of pruritus, erythema, scaling and oedema in a subject suffering from eczemas or having eczema, such as preferably eczemas selected from atopic dermatitis, hand eczema, infantile eczema, child hood eczema, adult eczema, keratosis pilaris, ichthyosis vulgaris, hand and foot dermatitis, keratoconus, pompholyx, discoid eczema, nummular eczema, allergic contact dermatitis, irritant contact dermatitis, over-treatment dermatitis and/or hand eczema.

In other embodiments, the treatment of pruritus is in a subject having asteatotic eczema, stasis dermatitis, lichen simplex chronicus, prurigo nodularis, seborrheic dermatitis, seborrhea or psoriasis.

The term "pruritus" is interchangeable with the term "itch" and defines a well known sensory state associated with the desire to scratch. The sensory state associated with pruritus is different from that of pain although pruritus and pain can be produced by a variety of chemical, mechanical, thermal or electrical stimuli. Itch and pain differ in that (1) itch, unlike pain, can only be evoked from the superficial layers of skin, mucosa, and conjunctiva, and (2) itch and pain usually do not occur simultaneously from the same skin region. For example, the application of histamine to skin produces itch but not pain. Furthermore, itch and pain are treated by different pharmacologically principles in that itch appears to be insensitive to opiate and non-steroidal anti-inflammatory drug (NSAID) treatment, both of which are effective in treating pain. Finally, itch occurs only in the skin; pain arises from deeper structures as well. Pruritus may be localised in various well defined areas of the skin, such as skin of the ankle, wrest, lips, hands, chest and the like, or it might be general pruritus not localised in a particular part of the skin.

It is to be understood that dermatological causes of pruritus often relates to allergic reactions, such a type-I or type-IV allergies in skin. Still other embodiments may refer to the treatment or prevention of eczemas which do not typically involve a hypersensitivity reaction, such as asteatotic eczema including senile pruritus, stasis dermatitis, lichen simplex chronicus, prurigo nodularis, psoriasis, seborrheic dermatitis and seborrhea.

Still another aspect of the invention relates to a method for detecting drug agents that are useful in the treatment of an inflammatory dermatological disease, such as particularly eczemas, the method comprises i) screening drug agents for their inhibitory activity towards at least two or three of the enzymes Protein tyrosine kinase Syk, Protein tyrosine kinase ZAP-70 and PDE-IV; and administering to a subject in need thereof one single drug agent or a combination of drug agents that act as

1) an inhibitor of Protein tyrosine kinase Syk and Protein tyrosine kinase ZAP-70;
2) an inhibitor of Protein tyrosine kinase Syk and PDE-IV enzyme;
3) an inhibitor of Protein tyrosine kinase ZAP-70 and PDE-IV enzyme; or
4) an inhibitor of Protein tyrosine kinase Syk and Protein tyrosine kinase ZAP-70 and PDE-IV enzyme.

*Oxaprozin and closely related compounds*

As mentioned, in currently interesting embodiments of the invention, the inhibitor of the Protein Kinase SYK and the Protein Kinase ZAP-70 and/or PDE-IV enzyme is Oxaprozin or a salt thereof.

Oxaprozin is chemically designated 4,5-diphenyl-2-oxazole-propionic acid, and has the following chemical structure:

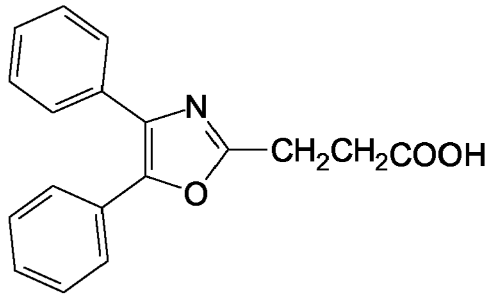

It should be understood that in a preferred embodiment of the invention, Oxaprozin is applied in un-derivatized form or as a pharmaceutically acceptable salt thereof or as a hydrolysable ester or amide.

It is anticipated that the novel pharmaco-dynamic effect of Oxaprozin is also exhibited by structurally closely related compounds and bioisosters of Oxaprozin. Several of such compounds with anti-inflammatory effect have been described in the patent literature. General derivatives as well as the manufacturing thereof are described in US 3,578,671. Specific derivatives and the manufacturing thereof are described in US 5,380,738 (4-fluorophenyl and 4-methylsulfonylphenyl), US 4,659,728 (hydroxy substituted derivatives), US 6,090,834 (sulfonyl derivatives), US 3,506679 (4,5-diarylthiazol derivatives).

Therefore as used herein, the term "closely related compound" includes compounds with the general formula I:

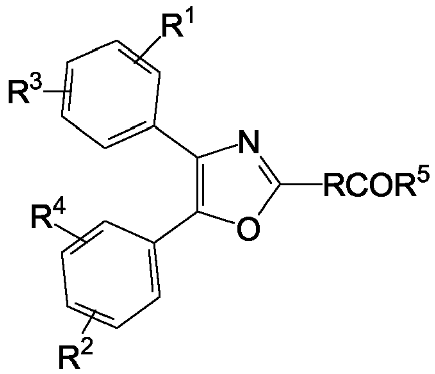

I

and bioisosters thereof.

Derivatives of Oxaprozin include various lengths of the R carbon chain, implying that the propionic acid of the Oxaprozin molecule be replaced with acetic acid or butyric acid. The acid group may be derivatized into amides and esters, preferably into hydrolysable amides and esters, that upon administration to a human or animal, are capable of providing (directly or indirectly) Oxaprozin or an active metabolite or residue thereof or a derivative of Oxaprozin as defined herein. Furthermore, the R chain and the two phenyl rings may be subject to substitution by replacing one or more hydrogen(s) with substituents defined herein. Finally, the term "derivatives thereof" include bioisosters where the oxygen of the oxazole ring is replaced with sulfur (S) to provide a thiazole ring. A bioisoster may also be provided by replacing the carboxylic acid group with a thioacid, optionally in the form of a thioester by properly selecting $R^5$.

As used herein, the group R primarily defines straight chained or branched, saturated or unsaturated aliphatic carbon chain containing 2 carbon atoms connected in one end to the 2-position of the oxazole ring and in the other end to $COR^5$. Thus, the R chain in combination with the $COR^5$-group forms n-propionic, iso-propionic and propionenic acids, esters and amides thereof. In closely related embodiments, the group R defines a straight-chained or branched, saturated or unsaturated aliphatic radical containing 1 or 3 carbon atoms. Thus, acetic, acrylic and butyric acids, esters and amides thereof are also anticipated.

Thus, R may be selected from $C_{1-3}$-alkyl, $C_{2-3}$-alkenyl, and $C_{2-3}$-alkynyl. The groups $C_{1-3}$-alkyl, $C_{2-3}$-alkenyl, and $C_{2-3}$-alkynyl may optionally be derivatized by substitution of one hydrogen atom with cyano (CN), halogen (Br, Cl, F, I), hydroxy (OH), amino ($NH_2$), or nitro ($NO_2$).

In preferred embodiments, together with the $COR^5$, the R group constitutes a free acid side chain (where $R^5$ is hydroxy (OH) or sulfhydryl (SH) such as in the form of acetic acid, acrylic acid, propionic acid, butyric acid including unsaturated, geometric and stereo isomers thereof. However, in other embodiments, the free acid ($R^5$ is OH or SH) is converted into suitable esters ($R^5$ is $OR^6$ or $SR^6$) or amides ($R^5$ is amino ($NH_2$), primary amino (NHR') or secondary amino (NR'R")).

Thus, $R^5$ may be selected from OH, $OR^6$, $NH_2$, NHR', NR'R", SH or $SR^6$.

$R^6$ designates a radical selected from $C_{1-6}$-alkyl, $C_{2-6}$-alkenyl and aryl.

R' and R" define the same or a different group selected from $C_{1-6}$-alkyl and $C_{2-6}$-alkenyl.

The term "aryl" means phenyl, mono- or di-substituted phenyl wherein one or two hydrogens have been replaced by substituents selected from $C_{1-6}$-alkyl, $C_{2-6}$-alkenyl, $C_{2-6}$-alkynyl, $C_{1-6}$-alkoxyl, carboxy (CO), carboxyderivative (COR'), carboxyaldehyde (CHO), carboxylic acid (COOH), carboxylderivative (COOR') cyano (CN), halogen (Br, Cl, F, I), hydroxy (OH), amino ($NH_2$), primary amino (NHR'), secondary amino (NR'R") and nitro ($NO_2$). Preferably, the term "aryl" means phenyl or mono-substituted phenyl wherein one hydrogen has been replaced by substituents selected from $C_{1-6}$-alkyl, $C_{2-6}$-alkenyl, $C_{1-6}$-alkoxyl, CO, CHO, CN, halogen, OH, $NH_2$ and $NO_2$.

The terms "$R^1$, $R^2$, $R^3$ and $R^4$" are meant to define substituents of the phenyl rings of formula I so as to define un-substituted, mono-substituted or di-substituted phenyl rings, wherein $R^1$, $R^2$, $R^3$ and $R^4$ may be the same or different. The phrase "mono and di-substituted phenyl radicals" designates substitution of one or two hydrogen(s) in each phenyl ring with $R^1$ and/or $R^3$ in one ring and independently thereof with $R^2$ and/or $R^4$ in the second phenyl ring.

$R^1$ and $R^2$ independently designate radicals selected from hydrido, $C_{1-6}$-alkyl, $C_{2-6}$-alkenyl, $C_{2-6}$-alkynyl, $C_{1-6}$-alkoxyl, carboxy (CO), carboxyaldehyde (CHO), carboxylic acid (COOH) and a derivative thereof (CO-Me, CO-Et), cyano (CN), halogen (Br, Cl, F, I), hydroxy (OH), hydroxy derivative (OR'), amino ($NH_2$), primary amino (NHR'), secondary amino (NR'R"), nitro ($NO_2$), sulfonyl ($HSO_2$) and sulfonyl derivative ($R^7$-$SO_2$,), wherein $R^5$, $R^6$, R' and R" are as defined above. $R^7$ designates a substituent selected from $C_{1-6}$-alkyl, aryl, $NH_2$, NHR', NR'R", wherein aryl and R' and R" are as defined above.

$R^3$ and $R^4$ independently designate radicals selected from hydrido, $C_{1-6}$-alkyl, $C_{2-6}$-alkenyl, $C_{2-6}$-alkynyl, $C_{1-6}$-alkoxyl, CO, CHO, CO-Me, CO-Et, CN, $COR^5$, halogen, OH, OR', $NH_2$, NHR', NR'R" and $NO_2$, wherein $R^5$, $R^6$, R' and R" are as defined above. In a preferred embodiment, $R^3$ and $R^4$ independently designate radicals selected from hydrido, $C_{1-6}$-alkyl and $C_{2-6}$-alkenyl. In a still more preferred embodiment, $R^3$ and $R^4$ each designate hydrido.

In the present context $C_{1-3}$-alkyl is designated to define a straight-chained or branched carbon chain having from 1 to 3 carbon atoms and wherein the carbon chain is situated between the oxazole ring and the $COOR^5$ group, such as $-CH_2-$, $-CH_2CH_2-$ and $-CH_2CH_2CH_2-$, including isomers thereof. Likewise, $C_{2-3}$-alkenyl and $C_{2-3}$-alkynyl means an unsaturated aliphatic carbon chain containing 2 or 3 carbon atoms, such as -CHCH-, -CC-, $-CHCHCH_2-$, $-CCCH_2-$, including isomers thereof.

$C_{1-6}$-alkyl is meant to define saturated, straight chained or branched alkyl radicals containing the number of carbon atoms indicated, e.g. "1-6" means all alkyl radicals from methyl up to hexyl including all isomers thereof, e.g. iso-butenyl. Where applicable, the alkyl may be in cyclical form, such as cyclohexane.

$C_{2-6}$-alkenyl defines unsaturated straight chained or branched alkylene radicals containing the number of carbon atoms indicated e.g. 1- or 2-propenyl, 1-, 2- or 3-butenyl and the like and isomers thereof.

$C_{2-3}$-alkynyl defines unsaturated chained or branched alkynyl radicals containing the number of carbon atoms indicated, e.g. ethynyl, 1- or 2-propynyl and isomers thereof.

$C_{1-6}$-alkoxy means alkoxy radicals containing up to 6, preferably up to 4 carbon atoms, e.g. methoxy, ethoxy, propoxy etc.

The groups, $C_{1-6}$-alkyl and $C_{2-6}$-alkenyl, may optionally be mono-substituted with CN, CO, CHO, $COR^5$, halogen, OH, OR' $NH_2$, NHR', NR'R" and nitro, wherein $R^5$, $R^6$, R' and R" are as defined above.

The term halogen defines bromine, chlorine, fluorine and iodine. The term "hydrido" designates a single hydrogen atom (H).

The Oxaprozin derivatives of the present invention may contain asymmetric carbon atoms, and, therefore, the present invention may also include the individual diastereomers and enantiomers, which may be prepared or isolated by methods known to those skilled in the art.

As mentioned, in current interesting embodiments of the invention Oxaprozin or a pharmaceutically acceptable salt thereof is the therapeutically active ingredient.

In other interesting embodiments, the therapeutically active ingredient is a closely related compound according to formula I. In one group of embodiments designated A, R is $-CH_2-$. In another group of embodiments designated B, R is selected from $C_2$-alkyl, $C_2$-alkenyl, and $C_2$-alkynyl, such as $-CH_2CH_2-$, -CHCH-, -CC-. In still another group of embodiments (designated C), R is selected from $C_3$-alkyl, $C_3$-alkenyl, and $C_3$-alkynyl, such as $-CH_2CH_2CH_2-$, $-CHCHCH_2-$, $-CCCH_2-$ and geometric and stereo isomers thereof. In all such embodiments (A, B and C), R may be substituted in that one hydrogen atom is replaced with CN, halogen, OH, $NH_2$, $NO_2$, preferably with OH. Furthermore, in such embodiments, $R^1$, $R^2$, $R^3$, $R^4$, $R^5$, $R^6$, $R^7$, R' and R" are as defined above.

In further interesting embodiments of A, B and C (designated AA, BA, CA), $R^2$ and $R^4$ independently designates radicals selected from hydrido, $C_{1-6}$-alkyl, halogen, OH and OR' and $R^1$, $R^3$, $R^5$, $R^6$, $R^7$, R' and R" are as defined above.

In other further interesting embodiments of A, B and C (designated AB, BB and CB), $R^2$ and $R^4$ is hydrido and $R^1$, $R^3$, $R^5$, $R^6$, $R^7$, R' and R" are as defined above.

In further interesting embodiments of A, AA, AB, B, BA, BB, C, CA and CB, the groups $R^1$, $R^2$, $R^3$, $R^4$, $R^5$, $R^6$, $R^7$, R' and R" are as defined under the respective groups of embodiments, but the term "aryl" is meant to designate phenyl or mono substituted phenyl, wherein one hydrogen has been replaced by substituents selected from $C_{1-6}$-alkyl, $C_{2-6}$-alkenyl, $C_{2-6}$-alkynyl, $C_{1-6}$-alkoxy CN, CO, CHO, COOH, halogen, OH, $NH_2$, NHR', NR'R" and $NO_2$.

In still further interesting embodiments of A, AA, AB, B, BA, BB, C, CA and CB, the groups $R^1$, $R^2$, $R^3$, $R^4$, $R^5$, $R^6$, $R^7$, R' and R" are as defined under the respective groups of embodiments, but the term "aryl" is meant to designate phenyl or mono substituted phenyl, wherein one hydrogen has been replaced by substituents selected from $C_{1-6}$-alkyl, $C_{1-6}$-alkoxyl, CN, CHO, COOH, halogen, OH, $NH_2$, and $NO_2$.

In still further interesting embodiments of A, AA, AB, B, BA, BB, C, CA and CB, the groups $R^2$ and $R^4$ independently designates radicals selected from hydrido, $C_{1-6}$-alkyl, $C_{1-6}$-alkoxyl, CN, COOH, halogen, OH, $NH_2$, NHR, NR'R", $NO_2$, $HSO_2$, $R^7$-$SO_2$ and $R^1$, $R^3$, $R^5$, $R^6$, $R^7$ R' and R" are as defined under the respective embodiments. In such embodiments, the term "aryl" is meant to designate phenyl or mono substituted phenyl, wherein one hydrogen has been replaced by substituents selected from $C_{1-6}$-alkyl, $C_{1-6}$-alkoxyl, CN, CHO, COOH, halogen, OH, $NH_2$, and $NO_2$.

In still further interesting embodiments of A, AA, AB, B, BA, BB, C, CA and CB, the groups, $C_{1-6}$-alkyl, $C_{1-9}$-alkyl, $C_{2-6}$-alkenyl, $C_{2-9}$-alkenyl, $C_{2-6}$-alkynyl and $C_{2-9}$-alkynyl may optionally be mono-substituted with CN, halogen, OH, OR' $NH_2$, NHR', NR'R" and nitro and $R^1$, $R^2$, $R^3$, $R^4$, $R^5$, $R^6$, $R^7$ R' and R" are as defined under the respective embodiments. In such embodiments, the term "aryl" is meant to designate phenyl or mono substituted phenyl, wherein one hydrogen atom has been replaced by substituents selected from $C_{1-6}$-alkyl, $C_{1-6}$-alkoxyl, CN, CHO, COOH, halogen, OH, $NH_2$, and $NO_2$.

It should be understood that in still further interesting embodiments of all the ones mentioned above, R is $-CH_2$- or $C_2$-alkyl or $C_2$-alkenyl.

Typical examples of closely related compounds are:

4,5-diphenylthiazol-2-yl- propionic acid, optionally in the form of its ethyl or methyl ester;
4,5-diphenyloxazol-2-yl-acrylic acid;
4,5-diphenyloxazol-2-yl-acetic acid;
4,5-di-(4'-chlorophenyl)-oxazol-2-yl-propionic acid;
4,5-diphenyloxazol-2-yl)-propionamide;
4,5-diphenyloxazol-2-yl)-acrylic acid ethyl ester;
4-(4'-bromophenyl)-5-phenyloxazole-2-yl-propionic acid, optionally in the form of its methyl ester;
4-(4-hydroxyphenyl-5-phenyl-2-oxazole propanoic acid, optionally in the form of its ethyl or methyl ester;
4-(4-fluorophenyl)-5-[4-(methylsulfonyl)phenyl]-2-oxazolepropionic acid, optionally in the form of its methyl ester;
4-(4-fluorophenyl)-5-[4-(methylsulfonyl)-phenyl]-2-oxazoleacetic acid, optionally in the form of its ethyl ester;
4-(4-fluorophenyl)-5-[4-(methylsulfonyl)phenyl]-2-oxazolebutanoic acid, optionally in the form of its methyl ester;
4-(4-fluorophenyl)-5-(4-(methylsulfonyl)phenyl)-2-oxazolepropionic amide;
[4-(4-aminosulfonylphenyl)-5-(3,4-dichlorophenyl)]-2-oxazoleacetic acid, optionally in the form of its ethyl or methyl ester;
[4-(4-aminosulfonylphenyl)-5-(3-chloro-4-fluorophenyl)]-2-oxazoleacetic acid, optionally in the form of its ethyl or methyl ester;
[4-(4-aminosulfonylphenyl)-5-(3-fluoro-4-methoxyphenyl)]-2-oxazoleacetic acid, optionally in the form of its ethyl or methyl ester;
[4-(4-aminosulfonylphenyl)-5-(4-chlorophenyl)]-2-oxazoleacetic acid, optionally in the form of its ethyl or methyl ester;
[4-(4-aminosulfonylphenyl)-5-phenyl]-2-oxazolebutanoic acid, optionally in the form of its ethyl or methyl ester;
[4-(4-aminosulfonylphenyl)-5-phenyl]-2-oxazolepropanoic acid, optionally in the form of its ethyl or methyl ester;
[4-(4-aminosulfonylphenyl-5-(3,4-difluorophenyl)]-2-oxazoleacetic acid, optionally in the form of its ethyl or methyl ester;
[4-(4-methylsulfonylphenyl)-5-phenyl]-2-oxazoleacetic acid, optionally in the form of its ethyl or methyl ester;
[4-(4-methylsulfonylphenyl)-5-phenyl]-2-oxazolebutanoic acid, optionally in the form of its ethyl or methyl ester;
[4-(4-methylsulfonylphenyl)-5-phenyl]-2-oxazolepropanoic acid, optionally in the form of its ethyl or methyl ester;
4-[4-aminosulfonylphenyl)-5-(3-fluoro-4-methoxyphenyl)-2-oxazolyl].alpha.-bromoacetic acid, optionally in the form of its ethyl or methyl ester;
5-(4-nitrophenyl-4-phenyl-2-oxazole-2-yl propionic acid, optionally in the form of its ethyl or methyl ester;
5-(4'-fluorophenyl)-4-phenyloxazole-2-yl-propionic acid, optionally in the form of its methyl ester;
5-(4-hydroxyphenyl-4-phenyl-2-oxazole propanoic acid, optionally in the form of its ethyl or methyl ester;

5-(4-fluorophenyl)-4-[4-(methylsulfonyl) phenyl]-2-oxazolepropionic acid, optionally in the form of its ethyl or methyl ester;
[5-(4-aminosulfonylphenyl)-4-(4-chlorophenyl)]-2-oxazoleacetic acid, optionally in the form of its ethyl or methyl ester;
[5-(4-aminosulfonylphenyl)-4-phenyl]-2-oxazoleacetic acid, optionally in the form of its ethyl or methyl ester;
[5-(4-aminosulfonylphenyl)-4-phenyl]-2-oxazolebutanoic acid, optionally in the form of its ethyl or methyl ester;
[5-(4-aminosulfonylphenyl)-4-phenyl]-2-oxazolepropanoic acid, optionally in the form of its ethyl or methyl ester;
[5-(4-aminosulfonylphenyl)-4-phenyl]-2-oxazolepropionic acid, optionally in the form of its ethyl or methyl ester;
ethyl [4-(4-aminosulfonylphenyl)-5-(3-fluoro-4-methoxyphenyl)]-2-oxazoleacetate;
ethyl [4-(4-aminosulfonylphenyl)-5-phenyl]-2-oxazoleacetate;
ethyl [4-(4-aminosulfonylphenyl)-5-phenyl]-2-oxazolebutanoate;
ethyl [4-(4-aminosulfonylphenyl)-5-phenyl]-2-oxazolepropanoate;
ethyl [4-(4-methylsulfonylphenyl)-5-phenyl]-2-oxazoleacetate;
ethyl [4-(4-methylsulfonylphenyl)-5-phenyl]-2-oxazolebutanoate;
ethyl [4-(4-methylsulfonylphenyl)-5-phenyl]-2-oxazolepropanoate;
ethyl [5-(4-chlorophenyl) - 4-phenylthiazol] 2-yl propionic acid;
ethyl [5-(4-aminosulfonylphenyl)-4-phenyl]-2-oxazoleacetate;
ethyl [5-(4-aminosulfonylphenyl)-4-phenyl]-2-oxazolebutanoate;
ethyl [5-(4-aminosulfonylphenyl)-4-phenyl]-2-oxazolepropanoate;
methyl [4-(4-aminosulfonylphenyl)-5-phenyl]-2-oxazoleacetate;
methyl [4-(4-aminosulfonylphenyl)-5-phenyl]-2-oxazolebutanoate;
methyl [4-(4-aminosulfonylphenyl)-5-phenyl]-2-oxazolepropanoate;
methyl [4-(4-methylsulfonylphenyl)-5-phenyl]-2-oxazoleacetate;
methyl [4-(4-methylsulfonylphenyl)-5-phenyl]-2-oxazolebutanoate;
methyl [4-(4-methylsulfonylphenyl)-5-phenyl]-2-oxazolepropanoate;
methyl [5-(4-aminosulfonylphenyl)-4-phenyl]-2-oxazoleacetate;
methyl [5-(4-aminosulfonylphenyl)-4-phenyl]-2-oxazolebutanoate;
methyl [5-(4-aminosulfonylphenyl)-4-phenyl]-2-oxazolepropanoate.

In summary, Oxaprozin or a closely related compound is meant to include derivatives according to formula I, which include metabolites of Oxaprozin and suitable prodrugs thereof, bioisosters thereof and pharmaceutically acceptable salts thereof including a solvate of the salt. Typical examples of suitable esters are formiate, acetate, propionate, ascorbyl and benzoylate. Examples of metabolites of Oxaprozin are hydroxy substituted Oxaprozin, namely 5-(4-hydroxyphenyl)-4-phenyl-2-oxazolepropanoic acid, 4-(4-hydroxyphenyl)-5-phenyl-2-oxazolepropanoic acid and 4-(4-hydroxyphenyl)-5-(4-hydroxyphenyl)-2-oxazolepropanoic acid as described in US 4,659,728.

In considering providing dermatological formulations comprising high amounts of completely dissolved Oxaprozin or a closely related compound, the solubility of the actives needs to be improved. One means to that effect is to improve the solubility of Oxaprozin by forming a water-soluble salt of Oxaprozin or a closely related compound defined herein. Therefore, in some embodiments of the invention, optionally where $R^5$ is hydroxy (OH), the Oxaprozin itself or a closely related compound may be provided as a pharmaceutically acceptable water-soluble salt.

The term "water-soluble" is meant to define Oxaprozin or a derivative thereof modified in a manner resulting in much higher water-solubility than Oxaprozin itself, such as 5, 10, 20, 25, 40, 50, 75, 100, 200, 250, 400, 500, 750 and 1000 times higher. The solubility of Oxaprozin in water at 25°C is about 1.7 mg/ml. Therefore, a water-soluble modification of Oxaprozin or a derivative thereof is meant to denote a modification resulting in a solubility of the modified Oxaprozin or related compound in water at 25°C of at least 10, 20, 25, 30, 40 and 50 mg/ml, preferably at least 75, 100, 150, 200, 250 or even at least 300 mg/ml.

The term "closely related compound" is also meant to define a pharmaceutically acceptable salt of Oxaprozin or of the related compound. Thus, Oxaprozin and the closely related compound where $R^5$ is hydroxy (OH) may be provided in the form of a single salt, either as a base addition salt or as an acid addition salt, or in the form of a double salt in the event where both the free carboxylic acid and the nitrogen of the oxazole ring form a salt.

In the present context, the phrase "a pharmaceutically acceptable salt" encompasses a base addition salt derived from the reaction of the free propionic acid entity with inorganic bases (hydroxides) or organic bases or/and an acid addition salt derived from the reaction of the basic oxazole ring nitrogen with pharmaceutically acceptable acids. Thus, it might be understood that Oxaprozin may be provided in the form of an acid addition salt or a base addition salt or in the form of a double salt of mixed acid addition and base addition salt.

Examples of base addition salts encompass Na, K, Ca, Mg, Cu, Zn and Mn salts. Typically organic bases for use in the preparation of a base addition salt are primary, secondary or tertiary amines including alkylphenylamine, ammonia, 2-aminoethanol, aminopyrimidine, aminopyridine, arginine, benethamine, benzathine, betaine, caffeine, choline, deanol, diethanolamine, diethylamine, 2-diethylaminoethanol, 2-dimethylaminoethanol, ethylenediamine, N-ethylmorpholine, N-ethylpiperidine, glucamine, glucosamine, glycinol, hydrabamine, imidazol, isopropylamine, meglumine, methylglucamine, morpholine, piperazine, piperidine, procaine, purine, pyrrolidine, theobromine, thiamine, triethanolamine, triethylamine, trimethylamine, tripropylamine, tromethamine, spermidine, and the like). Furthermore, base addition salts may be derived from the reaction with natural amino acids such as with glycine, alanine, valine, leucine, isoleucine, norleucine, tyrosine, cystine, cysteine, methionine, proline, hydroxy proline, histidine, omithine, lysine, arginine, serine, threonine, and phenylalanine and with unnatural amino acids such as D-isomers or substituted amino acids; guanidine, substituted guanidine wherein the substituents are selected from nitro, amino, alkyl, alkenyl, alkynyl, ammonium or substituted ammonium salts and aluminum salts.

Examples of acid addition salts include those derived from inorganic acids like hydrochloric, hydrobromic, nitric, carbonic, monohydrogencarbonic, phosphoric, monohydrogenphosphoric, dihydrogenphosphoric, sulfuric, monohydrogensulfuric, hydriodic or phosphorous acids and the like, as well as the salts derived from relatively non-toxic organic acids like acetic, propionic, isobutyric, maleic, malonic, benzoic, succinic, suberic, fumaric, mandelic, phthalic, benzenesulfonic, p-tolylsulfonic, citric, tartaric, methanesulfonic, and the like. Also included are salts of amino acids such as arginate and the like, and salts of organic acids like glucuronic or galacturonic acids and the like.

Where Oxaprozin or a closely related compound is provided in the form of a salt it is meant to include a pharmaceutically acceptable solvate, which may be a hydrate (comprising from half a mole of $H_2O$ up to about 10 moles of $H_2O$ per mole of salt) or may comprise other solvents of crystallization such as alcohols.

As mentioned, the active ingredient of the invention may be administered to a subject through any route of administration resulting in either local presence of the agonist in skin or systemic uptake.

In currently interesting embodiments of the invention, the Oxaprozin or a closely related compound is administered topically to the skin of a subject. That is to say that Oxaprozin or a medicament thereof is preferably formulated for topical application to the skin, such as formulated in liquid or semi- solid form (including, for example, ointment, emulsion including microemulsions and liposomes, gel, liniment, powder or spray) or it may be provided in combination with a "finite" carrier, for example, a non-spreading material that retains its form, including, for example, a patch, bioadhesive, dressing or bandage. The Oxaprozin or a closely related compound may be formulated in aqueous or non-aqueous form, such as a solution, emulsion, dispersion, suspension or ointment.

Topical administration refers to the application of a dermatological composition comprising Oxaprozin or a closely related compound in a concentration of 0.01 - 50.0 % (w/w). The amount of dermatological composition applied depends on the duration of the treatment, the condition to be treated and the formulation. In a preferred embodiment of the invention the concentration of Oxaprozin or a closely related compound in the dermatological composition is 0.1 - 20.0 % (w/w). In an even more preferred embodiment of the invention the concentration in the formulation is 0.5 - 10.0 % (w/w).

In considering applying more effective amounts of the active compounds of the invention, a medicament, such as a dermatological formulation, and methods of topical administration of a therapeutically effective amount should comprise Oxaprozin or a closely related compound in an amount of at least 0.5% by weight, more preferably of at least 1% by weight, even more preferably of at least 1.5% by weight, still more preferably of at least 2 % by weight, such as about 2.5% by weight, about 3% by weight, about 3.5% by weight, about 4% by weight, about 4.5% by weight, about 5% by weight, about 5.5% by weight, about 6% by weight or about 7% by weight.

Although Oxaprozin or a closely related compound is well tolerated in skin, it may be considered to apply amounts of the actives that secure safe treatment. Therefore, a medicament, such as a dermatological formulation, and methods of topical administration of a therapeutically effective amount should comprise Oxaprozin or a closely related compound in an amount less than 7% by weight, more preferably less than 6.5 % by weight, even more preferably less than 6% by weight, still more preferably less than 5.5 % by weight, even still more preferably less than 5% by weight, such as less than 4.5% by weight, such as less than 4% by weight, or such as less than 3.5% by weight.

Accordingly, in preferred embodiments of the invention, a medicament, such as a dermatological formulation, and methods of topical administration of a therapeutically effective amount should comprise Oxaprozin or a closely related compound or a salt thereof in an amount ranging between 0.5 and 10% by weight, such as between 0.5 and 8% by weight, preferably between 0.5 and 7% by weight, such as between 0.5 and 6% by weight, between 0.5 and 5.5 % by weight, between 0.5 and 5% by weight, between 0.5 and 4.5% by weight, between 0.5 and 4% by weight, between 0.5 and 3.5% by weight, such as between 0.5 and 3% by weight. In still more preferred embodiments of the invention, a medicament, such as a dermatological formulation, and methods of topical administration of a therapeutically effective amount should comprise Oxaprozin or a closely related compound or a salt thereof in an amount ranging between 1 and 7% by weight, preferably between 1 and 6.5% by weight, such as between 1 and 6% by weight, between 1 and 5.5 % by weight, between 1 and 5% by weight, between 1 and 4.5% by weight, between 1 and 4% by weight, between 1 and 3.5% by weight, such as between 1 and 3% by weight. In still more preferred embodiments of the invention, a medicament, such as a dermatological formulation, and methods of topical administration a therapeutically effective amount should comprise Oxaprozin or a closely related compound or a salt thereof in an amount ranging between 1.5 and 7% by weight, preferably between 1.5 and 6.5% by weight, such as between 1.5 and 6% by weight, 1.5 and 5.5 %

by weight, 1.5 and 5% by weight, 1.5 and 4.5% by weight, 1.5 and 4% by weight, 1.5 and 3.5% by weight, such as 1.5 and 3% by weight.

In currently interesting embodiments of the invention, a medicament, such as a dermatological formulation, and methods of topical administration of a therapeutically effective amount should comprise Oxaprozin or a closely related compound in an amount of about 1%, of about 1.5%, of about 2%, of about 2.5%, of about 3%, of about 3.5%, of about 4% by weight, of about 4.5% weight, of about 5% by weight or of about 6% by weight, preferably 2.5%; 3%, 3.5% or 4% by weight.

It should be understood, that in preferred uses and methods of the invention, Oxaprozin or the closely related compound is the sole therapeutically active ingredient.

However, in other uses and methods, Oxaprozin or a closely related compound or a salt thereof is administered together with a dermatological treatment agent. This may be an effective treatment for dermatological diseases and in preferred embodiments the use of the two agents in combination is superior to the results that would be expected based on the use of either agent alone. For example, the combination therapy is effective for lowering the dosages of conventional dermatological agents that are normally prescribed as a mono-therapy. The administration of lower dosages of conventional treatment agents provides a reduction in side effects corresponding to such conventional agents.

Therefore, uses and methods of the invention further comprising the administration of a dermatological treatment agent.

Typical examples of dermatological treatment agents are antihistamines, anti-bacterial agents, anti-fungal agents, anti-pruritus agents, anti-viral agents, agents for combating parasites, steroidal anti-inflammatory agents, non-steroidal anti- inflammatory agents, anaesthetic agents, keratolytic agents, agents for combating free radicals, metal chelating agents, antidandruff agents, anti-acne vulgaris agents, substance P or bradykinin antagonists or NO-synthase inhibitors.

The invention is further described by the examples.

Example 1 describes a typical formulation of a dermatological composition of Oxaprozin in the form of its water-soluble salt (mono-ethanolamine salt) and the formation of the water-soluble salt of Oxaprozin.

Examples 2, 3 and 4 demonstrate the beneficial effect of topical application of Oxaprozin (as a water-soluble salt) in treating pruritus associated with contact dermatitis, atopic dermatitis, insect bite inflammation and psoriasis, respectively.

Example 5 demonstrates the new pharmacological properties of Oxaprozin, which could not be demonstrated for Bufexamac that only inhibits the PDE-IV enzyme.

Example 6 demonstrates the significant effect of Oxaprozin in preventing and treating experimental contact dermatitis in a dose related manner and with stronger effect than observed with betamethasone 17-valerate.

Example 7 demonstrates that Oxaprozin is able to inhibition the formation of ear oedema in an experimental contact dermatitis model, but that no effect could be observed for Bufexamac.

Example 8 demonstrates that Oxaprozin are safe when applied topically to skin and do not cause sensitization reactions, photo toxicity or acute dermal irritation even when applied in high dose.

Example 9 demonstrates that an emulsion of Oxaprozin (Example 1) has good cutaneous tolerance even when applied consecutively in a concentration of 5% for 28 days.

Examples 10 and 11 refer to the clinical assessment of the effect of Oxaprozin in the treatment of hand eczema and contact dermatitis, respectively.

## EXAMPLES

### Example 1

A topical pharmaceutical composition according to the invention was prepared by dissolving 2.5% or 5.0% of the monoethanolamine salt of Oxaprozin in the water phase of the topical emulsion with the following composition (w/w):

**Hydrophobic phase**

| | |
|---|---|
| Tween 80™ (Polyoxyethylene sorbitan monooleate) | 1% |
| Span 60™ (emulsifier of the sorbitan ester type) | 2% |
| Medium chain triglycerides (MCT) | 20% |
| Petrolatum, white | 10% |
| Paraffin, light | 10% |
| Cetanol | 4% |

**Hydrophilic phase**

| | |
|---|---|
| Oxaprozin monoethanolamine salt | 2.5% |
| Water | 42.5% |
| Xanthan gum | 0.5% |
| Glycerol | 2 % |
| Propylenglycol | 2% |
| Benzylalcohol | 0.5% |

The emulsion was prepared by first heating the lipophilic phase and some of the hydrophilic phase (xanthan gum and water) to 70 degrees Celsius, and mixing them. The remaining hydrophilic phase is heated to 50°C and added subsequently cooling them under agitation.

The monoethanolamine salt was prepared according to the following advantageous method:

10.0 g Oxaprozin was dissolved in 230 ml ethyl acetate under mild heating.
2.3 g of monoethanolamine was dissolved in 30 ml ethyl acetate and added to the Oxaprozin solution under agitation. After a few seconds a significant precipitation could be observed. The solution was allowed to cool for 60 minutes and the salt was collected by filtration and dried.

**Example 2**

A 71 year old male subject had been suffering from irritant contact dermatitis for more than 5 years. The dermatitis was usually situated on the legs. The symptoms of the dermatitis was erythema, scaling and significant pruritus. During the last 5 years the subject had regularly been treated with strong topical steroids with a relatively good therapeutic effect on the erythema, but with no short term effect on the pruritus. During an aggravation of the dermatitis associated with a strong itch, the subject initiated a treatment with the emulsion according to example 1 containing 5.0% of the monoethanolamine salt of Oxaprozin. The subject experienced an immediate and complete alleviation of the pruritus 20 minutes after application of the emulsion of example 1. To maintain this level of efficacy, the subject had to reapply the emulsion three times daily the first day and twice daily during the next two weeks, where the erythema and scaling were gradually reduced. After 14 days of treatment the erythema had completely gone and the treatment was stopped. Two weeks later the erythema had still not reappeared. This indicates a surprisingly good therapeutic effect not only on the pruritus, but also on the underlying disease.

A 3½ year old female had been suffering from atopic dermatitis for at least 2 years. The dermatitis was present in the face and on more than 30% of the body and was characterised by erythema and extensive pruritus. The subject had periodically been treated with hydrocortisone ointment or pimecrolimus cream with some effect on the erythema, but with no short term effect on the pruritus.
During an aggravation of the dermatitis with extensive pruritus, the subject was treated with the emulsion according to example 1 containing 2.5% of the monoethanolamine salt of Oxaprozin. 15 minutes after application of the emulsion, the subject experienced a complete alleviation of the pruritus, which lasted 8 hours. During the next week the treatment was repeated when needed, 1-3 times daily and every time a complete recovery from pruritus was observed. During the week of treatment a significant improvement of erythema was also observed indicating a surprisingly good therapeutic effect not only on the pruritus, but also on the underlying disease.

**Example 3**

A 37 year old female, which previously had experienced insect bite inflammation in skin with pruritus and oedema as predominant symptoms, was treated with the emulsion according to example 1 containing 2.5% of the monoethanolamine salt of Oxaprozin following an insect bite by a mosquito. This treatment completely alleviated the pruritus after 20 minutes of application of the emulsion and the oedema disappeared overnight.
Contrarily, treatment of previous mosquito attacks with hydrocortisone ointment did not satisfactorily reduce pruritus and oedema.

**Example 4**

A 32 year old male subject had been suffering from plaque psoriasis for more than two years. The disease was apparent on the elbows with erythema, scaling and significant pruritus. During an aggravation of the symptoms the subject initiated a week of twice daily treatment with the emulsion according to claim 1 containing 5.0% of the monoethanolamine salt of Oxaprozin. The subject experienced an immediate and significant relief of pruritus after the first treatment. This level of efficacy was maintained for the entire week of treatment. Furthermore a significant reduction of erythema and scaling was observed. Again this indicated a surprisingly good therapeutic effect not only on the pruritus, but also on the underlying disease.

**Example 5**

The anti-inflammatory potential of Oxaprozin was determined by evaluating the inhibitory activity of Oxaprozin against the enzymes *Phosphodiesterase PDEIV, Protein Tyrosine Kinase SYK and Protein Tyrosine kinase ZA70 (ZAP-70).* The enzyme assays were conducted by MSD Pharma Services.
The following concentration of Oxaprozin (as the monoethanolamine salt) resulted in 50% inhibition of the following enzymes ($IC_{50}$);

| **Enzyme** | **MDS Pharma Service No:** | **$IC_{50}$** |
|---|---|---|
| Phosphodiesterase PDE IV | 154000 | 22 μM |
| Protein Tyrosine Kinase, SYK | 155761 | 28 μM |
| Protein Tyrosine Kinase, ZA70 (ZAP-70) | 155987 | 38 μM |

In comparison, Bufexamac only exhibits inhibitory effect on the PDE-IV enzyme.

**Example 6**

Screening for Anti-inflammatory Effect in the Oxazolone induced Mouse Ear Oedema Assay.
The anti-inflammatory activity of Oxaprozin was assessed by topical administration of Oxaprozin to oxazolone induced ear inflammation in mice. This screening method is commonly employed for screening and evaluation of anti-inflammatory drugs, in particular with respect to the inflammation seen in contact dermatitis. Betamethasone-17 valerate was used as the positive control.
Oxaprozin in the form of the monoethanolamine salt was administered topically as a dilution in acetone in a quantity of 250-1000μg/ear. Betamethason was administered topically in quantities of 20μg/ear. Betamethason was applied in the commercial form Celeston valerate ® 0.1%.

<u>Test Procedure</u>

Day 0

All groups were immunised with 20 μl oxazolone, 1.6% in ethanol 96% (w/v) on the left and the right ear.

Day 7

The ear thickness of all mice on both the left and right side was measured with an electronic measuring gauge. All groups were challenged with 20 μl oxazolone (1.6% in ethanol 96% (w/v)) on the left ear and the right ear. Vehicle (acetone) or test article solutions were administered 20 minutes before and 20 minutes after oxazolone challenge.

Day 8

24 hours after oxazolone challenge the ear thickness of all mice was measured with an electronic measuring gauge.

The groups, doses and animal numbers will be as follows:

| Group | Drug | Dose | Animal numbers |
|---|---|---|---|
| 1 | Vehicle, acetone | - | 1-10 |
| 2 | Monoethanolamine Oxaprozin | 1000μg/ear | 11-20 |
| 3 | Monoethanolamine Oxaprozin | 500μg/ear | 21-30 |
| 4 | Monoethanolamine Oxaprozin | 250μg/ear | 31-40 |
| 5 | Betamethasone 17-valerate | 20μg/ear | 41-50 |

Mean thickness of the ears and standard deviations were calculated. Ear swelling was calculated as the difference between the ear thickness day 7 and day 8. Percent inhibition of the ear swelling was assessed as the difference between the mean ear swelling of group 1 and the mean ear swelling of groups 2 to 5 expressed in percent.

Statistics

Differences in ear swelling between the vehicle treated group and the other groups were tested for significance employing a non-parametric statistical method of analysis, the Mann-Whitney U test. The required level of significance was $p<0.05$.

Results

The oxazolone challenge caused an inflammation in the ears, which was significant in the vehicle treated group after 24 hours since the ears were swollen and bright red. The test articles to some extent prevented the reaction. No adverse reactions to any of the test articles were observed.

Ear swelling

The various concentrations of the test articles inhibited the ear swelling as shown in the table below:

| Drug | Dose | % inhibition of ear swelling | Mann-Whitney U test |
|---|---|---|---|
| Vehicle, acetone | - | - | - |
| Monoethanolamine Oxaprozin | 1000μg/ear | 95 | P<0.001 |
| Monoethanolamine Oxaprozin | 500μg/ear | 77 | P<0.001 |
| Monoethanolamine Oxaprozin | 250μg/ear | 53 | P<0.001 |
| Betamethasone 17-valerate | 20μg/ear | 66 | P<0.001 |

Conclusion

The Oxaprozin monoethanolamine salt of the invention displayed a dose dependent and highly significant inhibition of ear swelling. The inhibition observed with the highest dose was significantly stronger than the inhibition obtained with Betamethasone 17-valerate in its clinically used dose level.
The data indicate that the Oxaprozin monoethanolamine salt of the invention has a strong suppressing effect on contact dermatitis.

**Example 7**

Comparison of Oxaprozin and Bufexamac in the Oxazolone induced Mouse Ear Oedema Assay.

The anti-inflammatory activity of Oxaprozin in comparison to Bufexamac was assessed using the same test method as described in Example 6. Both test articles were applied in a dose of 500 μg/ear and dissolved in 96% ethanol. Betamethason, as the positive control was administered topically in quantities of 20μg/ear.

Results:

Oxaprozin showed a statistically significant inhibition of the formation of ear oedema, but Bufexamac did not inhibit the formation of ear oedema at all.

**Example 8**

**Acute dermal irritation**

A sample of oxaprozin as the monoethanolamine salt was prepared in two concentrations (2.5% and 5% by weight) by dilution with water and tested for acute dermal irritation.

Procedure:

The sample was applied at a dose of 0.5 mL, on an undamaged skin area of the right flank of each animal. The patch was secured in position with a strip of surgical adhesive tape.
On the left flank an untreated area served as the control.
The skin reactions were evaluated after 1 hour and then after 24, 48 and 72 hours following removal of the patch according to the following grading scale:

Grading scales:

Erythema (0: No erythema, 1: Slight(barelyperceptible)erythema, 2: Definite erythema, 3: Moderate to severe erythema, 4: Severe erythema (purpie) with formation of eschars (deep lesions) preventing erythema from being grading).
Oedema (0: No oedema, 1: Very slight (barely perceptible) oedema, 2: Slight oedema (contour clearly defined), 3: Moderate oedema (thickness), 4: Severe oedema (thickness greater than 1 mm, surface larger than zone of application)

Results:

With respect to Oxaprozin (2.5% by weight), no cutaneous reactions (erythema and oedema) were observed irrespective of the examination time.
With respect to Oxaprozin (5% by weight), only a slight erythema on the treated area at the reading time 1 hour was observed. This reaction was totally reversible between the 2nd and the 3rd day of the test.

**Phototoxicity:**

Test for phototoxicity is carried out to evaluate the risk of cutaneous reactions on the guinea pig following exposure to ultraviolet radiation.

Procedure:

Oxaprozin (supplied as its monoethanolamine salt) was diluted in water to produce solutions containing either 2.5% or 5% by weight of the Oxaprozin salt. The solution was applied at a dose of 0.5 mL over the whole right-hand flank of each guinea pig. Thirty minutes after the treatment, the animals were subjected to ultraviolet radiations (UV-B first and then UV-A).
The animals were irradiated with the irradiation source VLX 3W (Biotronic, Vilbert Lourmat) at the Maximal Non Erythemateous Dose (M.N.E.D) 7000 J/cm2 for UV-A and 150mJ/cm2for the UV-B.

Results:

A macroscopic evaluation of the cutaneous reactions (erythema and oedema) was conducted 24 and 48 hours after irradiation. No macroscopic cutaneous reaction was attributable to photo irritation as compared with the reactions noticed on the reference sites (8-Methoxypsoralen: positive reference and product alone: negative reference). Thus, Oxaprozin is not phototoxic.

**Skin Sensitization**

Test for skin sensitization is carried out according to the Magnusson and Kligman method (J. Invest. Dermatol. 1969. 52, 268-276) and in accordance with O.E.C.D. Guideline N° 406 of July 17th, 1992, and the test method B.6 of the 96/54 E.E.C Directive.

Procedure:

Oxaprozin (supplied as its monoethanolamine salt) was diluted in water to produce solutions containing 2.5% by weight of the Oxaprozin salt.

Albino guinea pigs of Dunkin-Hartley strain were exposed to the test item after an acclimatisation period of at least five days.

The Maximum Non Necrotizing Concentration (M.N.N.C.) was determined by injecting by intradermal route the following concentrations: 2.5%, 1.25%, 0.625%, 0.3125%, 0.1562% and 0.078% diluted in physiological saline solution.

Pre-Maximum Non Irritant Concentration (pre-M.N.I.C.) was determined by application of the test item under an occlusive dressing during 24 hours, at the following concentrations: 2.5%, 1.25%, 0.625%, 0.3125% diluted in physiological saline solution.

Maximum Non Irritant Concentration (M.N.I.C.) was determined by initially establishing an induction phase by intradermal injection with a physiological saline solution and by topical application of distilled water followed by a 18-day rest phase. In the challenge phase where the test item is under occlusive dressing for 24 hours, the test item was applied to the skin of the Albino guinea pigs at the following concentrations: 2.5%, 1.25%, 0.625%, 0.3125% diluted in physiological saline solution.

Results:

No macroscopic cutaneous reactions attributable to allergy was recorded during the examination following the removal of the occlusive dressing (challenge phase) from the animals of the treated group. No cutaneous intolerance reaction was recorded in animals from the negative control group. Thus, Oxaprozin monoethanolamine salt is found to not causing sensitization reactions.

**Example 9**

Cutaneous tolerance of an emulsion (Example 1) containing Oxaprozin (as the monoethanolamine salt) 2.5% and 5% by weight, respectively, was tested by daily application at a dose of 2 ml per animal per day for 28 consecutive days on undamaged skin of rabbits.

Macroscopic cutaneous examinations were performed daily during the 28 days just before the daily application of the emulsion. Skin erythema, oedema, dryness, elasticity and skin fold thickness were assessed.

The results obtained showed slight erythema and oedema after some days of treatment but was totally reversed before the 19th and 10th day, respectively. Dryness was noted too in the beginning of the treatment and there was also observed slight skin fold thickening. The investigator concluded that the emulsion, both in 2.5% and 5% concentration, presented good dermal cutaneous tolerance after repeated application for 28 days.

**Example 10**

The efficacy of Oxaprozin or a related compound to treat and prevent hand eczema can be tested in a blinded treatment with the study preparation or vehicle twice daily for 4 weeks in 2 treatment groups with chronic hand dermatitis. Half of the patients will be treated with a cream formulation of Oxaprozin, e.g. Oxaprozin monoethanolamine salt in a concentration of 2.5% and the second half with the cream vehicle. Clinical assessment will be performed on day 1 prior to the first treatment (baseline) and following 1, 2, 3 and 4 weeks of treatment. Additionally the patients will answer a questionnaire for determination of the Dermatology Life Quality Index, a patients global assessment will be performed. The dosage to be applied is approximately 25 mg per day and the total dosage amounts to approximately 700 mg.

The clinical assessment can be done according to the HECSI scoring system that is an objective and accurate assessment of the severity of hand eczema. It incorporates both the extent and the intensity of the disease. Each hand is divided into five areas (fingertips, fingers (except the tips), palms, back of hand and wrists). For each of these areas the intensity of each of the clinical signs erythema, induration/papulation, vesicles, fissuring, scaling and edema is graded on the following four point scale:

0 = no skin changes, 1 = mild disease, 2 = moderate, 3 = severe

For each location (total of both hands) a score from 0 to 4 is given for the extent of clinical symptoms with respect to the percentual affected area:

0 = 0 %, 1 = 1 – 25 %, 2 = 26 – 50 %, 3 = 51 – 75 %, 4 = 76 – 100 %

Finally the score given for the extent at each location is multiplied by the sum of the intensity of each clinical feature (Erythema (E), Infiltration/papulation (I), Vesicles (V), Fissures (F), Scaling (S), Edema (O).

**Example 11**

The efficacy of Oxaprozin or a related compound to treat and prevent contact dermatitis can be assessed clinically in humans in a randomized, controlled double-blind study using test persons with known nickel allergy and with healthy skin in the test area. According to a standard procedure, at least three test fields of healthy skin located on the back are assigned to each test person, in which fields' allergy is provocated by application of nickel II sulfate vaseline and test medication is applied in order to test efficacy. The test medication can be a cream formulation of Oxaprozin, e.g. Oxaprozin monoethanolamine salt in a concentration of 2.5% or 5% where the daily dose applied to the test field is approximately 15 mg and 40 mg, respectively. As positive control can be used Betamethasone 17- valerate Cream 0.1%, where the daily dose applied is approximately 0.6 mg betamethasone/day. Furthermore, active ingredient-free vehicle is also tested. On day 1, study medication, positive control and vehicle is applied to test fields (= pretreatment) in that approximately 200 μl of each study preparation will be applied to the respective test fields, for example by using special test chambers (Finn Chambers®, Epitest Ltd. Oy, Finland, 18 mm inside in diameter.
On the consecutive day the pre-treated test fields will be treated with two concentrations of nickel II sulfate vaseline to induce an allergic reaction or with vaseline for 1 hours. The treatment with the different concentrations of nickel II sulfate vaseline will be performed in an smaller area in the middle of the defined pre-treated test fields using smaller test chambers, e.g. Finn Chambers®, Epitest Ltd. Oy, Finland, 12 mm inside in diameter. Approximately 30 μl nickel II sulfate vaseline or vaseline will be used. After this induction the efficacy of preventative treatment with the study preparations will be assessed. On study day 4 to 7 the test fields will be treated as described for day 1 with the study preparations once daily to assess the efficacy in the treatment of contact dermatitis. The extent of epidermal barrier impairment measured by TEWL, skin redness measured by chromametry and clinical skin condition evaluated by scoring will be determined. In addition photodocumentation will be performed. Clinical assessment will be performed according to the following score:
0 = no reaction, 1 = erythema, but no induration, 2 = erythema, induration, discrete papules possible, 3 = erythema, induration, papules, vesicle, 4 = erythema, induration, confluencing vesicle.

Transepidermal water loss (evaporimetry) is a widely used non-invasive method for evaluation of skin impairment. The epidermis of healthy intact skin represents a barrier that minimizes external water loss. Any impairment of this barrier results in an increase of permeability to water with a corresponding increase of TEWL. Increased TEWL values are to be expected in the lesional test fields in subjects with allergic reactions induced by the treatment with nickel II sulfate vaseline.

**Claims**

1. Use of Oxaprozin or a closely related compound or a pharmaceutically acceptable salt thereof for the preparation of a medicament formulated for topical administration to skin for the treatment of an inflammatory dermatological disease in a subject,
wherein the closely related compound is defined by the general formula I:

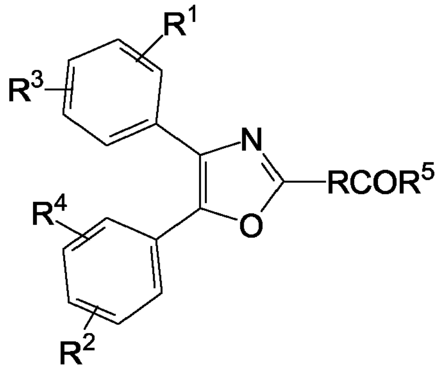

I

and wherein

R is selected from $C_{1-3}$-alkyl, $C_{2-3}$-alkenyl, and $C_{2-3}$-alkynyl and R is optionally derivatized by substitution of one hydrogen atom with CN, halogen OH, $NH_2$ and $NO_2$;

$R^1$ and $R^2$ independently designate radicals selected from hydrido, $C_{1-6}$-alkyl, $C_{2-6}$-alkenyl, $C_{2-6}$-alkynyl, $C_{1-6}$-alkoxyl, CO, CHO, CO-Me, CO-Et, CN, halogen, OH, OR', $NH_2$, NHR', NR'R", $NO_2$, $HSO_2$ and $R^7$-$SO_2$;

$R^3$ and $R^4$ independently designate radicals selected from hydrido, $C_{1-6}$-alkyl and $C_{2-6}$-alkenyl;

$R^5$ designate radicals selected from OH, $OR^6$, $NH_2$, NHR', NR'R", SH and $SR^6$;

$R^6$ designate radicals selected from $C_{1-6}$-alkyl, $C_{2-6}$-alkenyl and aryl;

$R^7$ designate radicals selected from $C_{1-6}$-alkyl, aryl, $NH_2$, NHR' and NR'R";

R' and R" designate the same or different group selected from $C_{1-6}$-alkyl and $C_{2-6}$-alkenyl; and

"aryl" means phenyl or mono-substituted phenyl wherein one hydrogen have been replaced by substituents selected from $C_{1-6}$-alkyl, $C_{2-6}$-alkenyl, $C_{1-6}$-alkoxyl, CO, CHO, CN, halogen, OH, $NH_2$ and $NO_2$;

and wherein the oxygen of the oxazole ring optionally is replaced with sulfur (S) to provide a thiazole ring.

**2.** Use of Oxaprozin or a closely related compound or a pharmaceutically acceptable salt thereof for the preparation of a medicament formulated for systemic administration for the treatment of an inflammatory dermatological disease in a subject, wherein the closely related compound is defined by the general formula I of claim 1 and wherein R, $R^1$, $R^2$, $R^3$, $R^4$, $R^5$, $R^6$, $R^7$, R', R", aryl and bioisoster are as defined in claim 1.

**3.** Use of Oxaprozin or a closely related compound or a pharmaceutically acceptable salt thereof for the preparation of a medicament formulated for topical administration to skin for the inhibition of at least two of the enzymes selected from the group consisting of an inhibitor of Protein tyrosine kinase Syk; Protein tyrosine kinase ZAP-70; and PDE-IV enzyme in a subject with an inflammatory dermatological disease, wherein the closely related compound is defined by the general formula I of claim 1 and wherein R, $R^1$, $R^2$, $R^3$, $R^4$, $R^5$, $R^6$, $R^7$, R', R", aryl and bioisoster are as defined in claim 1.

**4.** Use of Oxaprozin or a closely related compound or a pharmaceutically acceptable salt thereof for the preparation of a medicament formulated for systemic administration to skin for the inhibition of at least two of the enzymes selected from the group consisting of an inhibitor of Protein tyrosine kinase Syk; Protein tyrosine kinase ZAP-70; and PDE-IV enzyme in a subject with an inflammatory dermatological disease, wherein the closely related compound is defined by the general formula I of claim 1 and wherein R, $R^1$, $R^2$, $R^3$, $R^4$, $R^5$, $R^6$, $R^7$, R', R", aryl and bioisoster are as defined in claim 1.

**5.** Use of at least two inhibitors selected from the group consisting of an inhibitor of Protein tyrosine kinase Syk; Protein tyrosine kinase ZAP-70; and PDE-IV enzyme for the preparation of a medicament formulated for topical administration to skin for the treatment of an inflammatory dermatological disease in a subject, wherein the closely related compound is defined by the general formula I of claim 1 and wherein R, $R^1$, $R^2$, $R^3$, $R^4$, $R^5$, $R^6$, $R^7$ R', R", aryl and bioisoster are as defined in claim 1.

**6.** Use of at least two inhibitors selected from the group consisting of an inhibitor of Protein tyrosine kinase Syk; Protein tyrosine kinase ZAP-70; and PDE-IV enzyme for the preparation of a medicament formulated for systemic administration to skin for the treatment of an inflammatory dermatological disease in a subject, wherein the closely related

compound is defined by the general formula I of claim 1 and wherein R, $R^1$, $R^2$, $R^3$, $R^4$, $R^5$, $R^6$, $R^7$ R', R", aryl and bioisoster are as defined in claim 1.

**7.** The use according to any one of claims 1 or 2, wherein the treatment of the inflammatory dermatological disease comprises inhibition of one or more of the enzymes Protein tyrosine kinase Syk; Protein tyrosine kinase ZAP-70 and phosphodiesterase PDE-IV.

**8.** The use according to any one of claims 1 to 6, wherein the inflammatory dermatological disease is **characterised by** being associated with a hypersensitivity reaction in the skin.

**9.** The use according to any one of claims 1 to 6, wherein the inflammatory dermatological disease is **characterised by** being associated with a type-IV allergy reaction in the skin.

**10.** The use according to any one of claims 1 to 6, wherein the inflammatory dermatological disease is **characterised by** being associated with a type-I allergy reaction in the skin.

**11.** The use according to any one of claims 1 to 6, wherein the inflammatory dermatological disease is eczema.

**12.** The use according to claim 11, wherein the eczema is selected from the group consisting of atopic dermatitis, hand eczema, infantile eczema, child hood eczema, adult eczema, keratosis pilaris, ichthyosis vulgaris, hand and foot dermatitis, keratoconus, pompholyx, discoid eczema, nummular eczema, allergic contact dermatitis, irritant contact dermatitis, overtreatment dermatitis and hand eczema.

**13.** The use according to any one of claims 1 to 6, wherein the inflammatory dermatological disease is selected from the group consisting of asteatotic eczema, stasis dermatitis, lichen simplex chronicus, seborrheic dermatitis, seborrhea and psoriasis.

**14.** The use according to any one of claims 1 to 6, wherein the inflammatory dermatological disease is contact dermatitis.

**15.** The use according to any one of claims 1 to 6, wherein the inflammatory dermatological disease is allergic contact dermatitis.

**16.** The use according to any one of claims 1 to 6, wherein the inflammatory dermatological disease is irritant contact dermatitis.

**17.** The use according to any one of claims 1 to 6, wherein the inflammatory dermatological disease is hand eczema.

**18.** The use according to any one of claims 1 to 6, wherein the inflammatory dermatological disease is atopic dermatitis.

**19.** Use of Oxaprozin or a closely related compound or a pharmaceutically acceptable salt thereof for the preparation of a medicament formulated for topical administration to the skin for the alleviation, removal or prevention of one or more symptoms selected from the group consisting of pruritus, erythema, scaling and oedema in a subject having eczema, wherein the closely related compound is defined by the general formula I of claim 1 and wherein R, $R^1$, $R^2$, $R^3$, $R^4$, $R^5$, $R^6$, $R^7$, R', R", aryl and bioisoster are as defined in claim 1.

**20.** Use of Oxaprozin or a closely related compound or a pharmaceutically acceptable salt thereof for the preparation of a medicament formulated for systemic administration for the alleviation, removal or prevention of one or more symptoms selected from the group consisting of pruritus, erythema, scaling and oedema in a subject having eczema, wherein the closely related compound is defined by the general formula I of claim 1 and wherein R, $R^1$, $R^2$, $R^3$, $R^4$, $R^5$, $R^6$, $R^7$, R', R", aryl and bioisoster are as defined in claim 1.

**21.** The use according to any one of claims 19 or 20, wherein eczema is selected from the group consisting of atopic dermatitis, hand eczema, infantile eczema, child hood eczema, adult eczema, keratosis pilaris, ichthyosis vulgaris, hand and foot dermatitis, keratoconus, pompholyx, discoid eczema, nummular eczema, allergic contact dermatitis, irritant contact dermatitis and over-treatment dermatitis.

**22.** The use according to any of the preceding claims, wherein the medicament formulated for topical administration to skin comprises an amount of Oxaprozin or the closely related compound or a salt thereof ranging between 0.5%

and 10% by weight.

**23.** The use according to any one of the claims 1 to 21, wherein the medicament comprises an amount of the Oxaprozin or the closely related compound or a salt thereof of about 2.5% by weight.

**24.** The use according to any one of the claims 1 to 21, wherein the medicament comprises an amount of the Oxaprozin or the closely related compound or a salt thereof of about 5% by weight.

**25.** The use according to any of the preceding claims, wherein the Oxaprozin or a closely related compound is provided in the form of a water-soluble salt.

**26.** The use according to any one of the preceding claims, wherein the closely related compound is selected from the group consisting of

4,5-diphenylthiazol-2-yl- propionic acid, optionally in the form of its ethyl or methyl ester;
4,5-diphenyloxazol-2-yl-acrylic acid;
4,5-diphenyloxazol-2-yl-acetic acid;
4,5-di-(4'-chlorophenyl)-oxazol-2-yl-propionic acid;
4,5-diphenyloxazol-2-yl)-propionamide;
4,5-diphenyloxazol-2-yl)-acrylic acid ethyl ester;
4-(4'-bromophenyl)-5-phenyloxazole-2-yl-propionic acid, optionally in the form of its methyl ester;
4-(4-hydroxyphenyl-5-phenyl-2-oxazole propanoic acid, optionally in the form of its ethyl or methyl ester;
4-(4-fluorophenyl)-5-[4-(methylsulfonyl)phenyl]-2-oxazolepropionic acid, optionally in the form of its methyl ester;
4-(4-fluorophenyl)-5-[4-(methylsulfonyl)-phenyl]-2-oxazoleacetic acid, optionally in the form of its ethyl ester;
4-(4-fluorophenyl)-5-[4-(methylsulfonyl)phenyl]-2-oxazolebutanoic acid, optionally in the form of its methyl ester;
4-(4-fluorophenyl)-5-(4-(methylsulfonyl)phenyl)-2-oxazolepropionic amide;
[4-(4-aminosulfonylphenyl)-5-(3,4-dichlorophenyl)]-2-oxazoleacetic acid, optionally in the form of its ethyl or methyl ester;
[4-(4-aminosulfonylphenyl)-5-(3-chloro-4-fluorophenyl)]-2-oxazoleacetic acid, optionally in the form of its ethyl or methyl ester;
[4-(4-aminosulfonylphenyl)-5-(3-fluoro-4-methoxyphenyl)]-2-oxazoleacetic acid, optionally in the form of its ethyl or methyl ester;
[4-(4-aminosulfonylphenyl)-5-(4-chlorophenyl)]-2-oxazoleacetic acid, optionally in the form of its ethyl or methyl ester;
[4-(4-aminosulfonylphenyl)-5-phenyl]-2-oxazolebutanoic acid, optionally in the form of its ethyl or methyl ester;
[4-(4-aminosulfonylphenyl)-5-phenyl]-2-oxazolepropanoic acid, optionally in the form of its ethyl or methyl ester;
[4-(4-aminosulfonylphenyl-5-(3,4-difluorophenyl)]-2-oxazoleacetic acid, optionally in the form of its ethyl or methyl ester;
[4-(4-methylsulfonylphenyl)-5-phenyl]-2-oxazoleacetic acid, optionally in the form of its ethyl or methyl ester;
[4-(4-methylsulfonylphenyl)-5-phenyl]-2-oxazolebutanoic acid, optionally in the form of its ethyl or methyl ester;
[4-(4-methylsulfonylphenyl)-5-phenyl]-2-oxazolepropanoic acid, optionally in the form of its ethyl or methyl ester;
4-[4-aminosulfonylphenyl)-5-(3-fluoro-4-methoxyphenyl)-2-oxazolyl].alpha.-bromoacetic acid, optionally in the form of its ethyl or methyl ester;
5-(4-nitrophenyl-4-phenyl-2-oxazole-2-yl propionic acid, optionally in the form of its ethyl or methyl ester;
5-(4'-fluorophenyl)-4-phenyloxazole-2-yl-propionic acid, optionally in the form of its methyl ester;
5-(4-hydroxyphenyl-4-phenyl-2-oxazole propanoic acid, optionally in the form of its ethyl or methyl ester;
5-(4-fluorophenyl)-4-[4-(methylsulfonyl) phenyl]-2-oxazolepropionic acid, optionally in the form of its ethyl or methyl ester;
[5-(4-aminosulfonylphenyl)-4-(4-chlorophenyl)]-2-oxazoleacetic acid, optionally in the form of its ethyl or methyl ester;
[5-(4-aminosulfonylphenyl)-4-phenyl]-2-oxazoleacetic acid, optionally in the form of its ethyl or methyl ester;
[5-(4-aminosulfonylphenyl)-4-phenyl]-2-oxazolebutanoic acid, optionally in the form of its ethyl or methyl ester;
[5-(4-aminosulfonylphenyl)-4-phenyl]-2-oxazolepropanoic acid, optionally in the form of its ethyl or methyl ester;
[5-(4-aminosulfonylphenyl)-4-phenyl]-2-oxazolepropionic acid, optionally in the form of its ethyl or methyl ester;
ethyl [4-(4-aminosulfonylphenyl)-5-(3-fluoro-4-methoxyphenyl)]-2-oxazoleacetate;
ethyl [4-(4-aminosulfonylphenyl)-5-phenyl]-2-oxazoleacetate;
ethyl [4-(4-aminosulfonylphenyl)-5-phenyl]-2-oxazolebutanoate;

ethyl [4-(4-aminosulfonylphenyl)-5-phenyl]-2-oxazolepropanoate;
ethyl [4-(4-methylsulfonylphenyl)-5-phenyl]-2-oxazoleacetate;
ethyl [4-(4-methylsulfonylphenyl)-5-phenyl]-2-oxazolebutanoate;
ethyl [4-(4-methylsulfonylphenyl)-5-phenyl]-2-oxazolepropanoate;
ethyl [5-(4-chlorophenyl) - 4-phenylthiazol] 2-yl propionic acid;
ethyl [5-(4-aminosulfonylphenyl)-4-phenyl]-2-oxazoleacetate;
ethyl [5-(4-aminosulfonylphenyl)-4-phenyl]-2-oxazolebutanoate;
ethyl [5-(4-aminosulfonylphenyl)-4-phenyl]-2-oxazolepropanoate;
methyl [4-(4-aminosulfonylphenyl)-5-phenyl]-2-oxazoleacetate;
methyl [4-(4-aminosulfonylphenyl)-5-phenyl]-2-oxazolebutanoate;
methyl [4-(4-aminosulfonylphenyl)-5-phenyl]-2-oxazolepropanoate;
methyl [4-(4-methylsulfonylphenyl)-5-phenyl]-2-oxazoleacetate;
methyl [4-(4-methylsulfonylphenyl)-5-phenyl]-2-oxazolebutanoate;
methyl [4-(4-methylsulfonylphenyl)-5-phenyl]-2-oxazolepropanoate;
methyl [5-(4-aminosulfonylphenyl)-4-phenyl]-2-oxazoleacetate;
methyl [5-(4-aminosulfonylphenyl)-4-phenyl]-2-oxazolebutanoate;
methyl [5-(4-aminosulfonylphenyl)-4-phenyl]-2-oxazolepropanoate.

**27.** The use according to any one of the preceding claims, wherein the subject is a human, a dog, a cat or a horse.

European Patent Office

# EUROPEAN SEARCH REPORT

Application Number
EP 06 11 2009

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | WO 02/074290 A (AGIS INDUSTRIES LTD; ARKIN, MOSHE; ZIGHELBOIM, MARCEL; LAVON, ILANA;) 26 September 2002 (2002-09-26)<br>* abstract; claims 1,3,4 *<br>* page 1, paragraph 2 - page 2, paragraph 1 * | 1-27 | INV.<br>A61K31/421<br>A61K31/426<br>A61P17/00<br>A61P17/04<br>A61P17/06<br>A61P17/08 |
| Y | | 2,4,6,20 | |
| | ----- | | |
| X | US 2003/082226 A1 (SAMOUR CARLOS M ET AL) 1 May 2003 (2003-05-01)<br>* abstract *<br>* page 3, paragraph 69 - page 4, paragraph 71 * | 1-27 | |
| Y | | 2,4,6,20 | |
| | ----- | | |
| Y | ED. BY KATHLEEN PARFITT: "Martindale, The complete drug reference, 32nd Edition" 1999, PHARMACEUTICAL PRESS , LONDON , XP002381108<br>* page 71, column 2 * | 2,4,6,20 | |
| | ----- | | |
| | | | TECHNICAL FIELDS SEARCHED (IPC) |
| | | | A61K |

The present search report has been drawn up for all claims

| Place of search | Date of completion of the search | Examiner |
|---|---|---|
| Munich | 22 May 2006 | Nyeki, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

8

## ANNEX TO THE EUROPEAN SEARCH REPORT ON EUROPEAN PATENT APPLICATION NO. EP 06 11 2009

This annex lists the patent family members relating to the patent documents cited in the above-mentioned European search report.
The members are as contained in the European Patent Office EDP file on
The European Patent Office is in no way liable for these particulars which are merely given for the purpose of information.

22-05-2006

| Patent document cited in search report | Publication date | Patent family member(s) | Publication date |
|---|---|---|---|
| WO 02074290 A | 26-09-2002 | AU 2002237489 A1 | 03-10-2002 |
| | | CA 2440687 A1 | 26-09-2002 |
| | | EP 1414429 A2 | 06-05-2004 |
| | | US 2003039704 A1 | 27-02-2003 |
| US 2003082226 A1 | 01-05-2003 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2005014729 A
- WO 05009342 A
- JP 7316075 A
- US 2005232957 A1, Katz K
- WO 9735573 A2
- WO 9511017 A1
- WO 02074290 A
- US 6589 A
- US 950 A
- WO 9931073 A
- US 20030229090 A
- US 3578671 A
- US 53807384 B
- US 4659728 A
- US 6090834 A
- US 3506679 A


### Non-patent literature cited in the description


- **GREAVES MW.** Pharmacology and significance of nonsteroidal anti-inflammatory drugs in the treatment of skin diseases. *J Am Acad Dermatol,* April 1987, vol. 16 ((4)), 751-64
- **DALLEGRI ; FRANCO.** A review of the emerging profile of the anti-inflammatory drug oxaprozin. *Expert Opin Pharmacother,* May 2005, vol. 6 ((5)), 777-85
- **GREAVES MW.** Pharmacology and significance of nonsteroidal anti-inflammatory drugs in the treatment of skin diseases. *J Am Acad Dermatol,* April 1987, vol. 16 ((4)), 751-64
- **GOING SM ; GUYER BM ; JARVIE DR ; HUNTER JA.** Salicylic acid gel for scalp psoriasis. *Clin Exp Dermatol,* May 1986, vol. 11 ((3)), 260-2
- **THOMSEN JS ; SIMONSEN L ; BENFELDT E ; JENSEN SB ; SERUP J.** The effect of topically applied salicylic compounds on serotonin-induced scratching behaviour in hairless rats. *Exp Dermatol,* August 2002, vol. 11 ((4)), 370-5
- **LOWE NJ ; VIRGADAMO F ; STOUGHTON RB.** Anti-inflammatory properties of a prostaglandin antagonist, a corticosteroid and indomethacin in experimental contact dermatitis. *BrJ Dermatol,* April 1977, vol. 96 ((4)), 433-8
- **BLACK AK ; HENSBY CN ; GREAVES MW.** The effect of topical flurbiprofen on human skin inflammation [proceedings]. *Br J Clin Pharmacol,* January 1980, vol. 9 (1), 125P
- **HUMPHREYS F ; SPIRO J.** The effects of topical indomethacin and clobetasol propionate on post-cryotherapy inflammation. *BrJ Dermatol,* May 1995, vol. 132 ((5)), 762-5
- **GREEN CA ; SHUSTER S.** Lack of effect of topical indomethacin on psoriasis. *BrJ Clin Pharmacol,* September 1987, vol. 24 ((3)), 381-4
- **GEBHARDT M ; WOLLINA U.** Cutaneous side-effects of nonsteroidal anti-inflammatory drugs (NSAID). *Z Rheumatol,* November 1995, vol. 54 ((6)), 405-12
- **RIVERS JK ; MCLEAN DI.** An open study to assess the efficacy and safety of topical 3% diclofenac in a 2.5% hyaluronic acid gel for the treatment of actinic keratoses. *Arch Dermatol,* October 1997, vol. 133 ((10)), 1239-42
- **KAWAI S.** Cyclooxygenase selectivity and the risk of gastrointestinal complications of various non-steroidal anti-inflammatory drugs. A clinical consideration. *In Inflamma. Res,* 1998, vol. 2, S102-S106
- **SHICHIJO M, ; YAMAMOTO N ; TSUJISHITA H ; KIMATA M ; NAGAI H ; KOKUBO T.** Inhibition of syk activity and degranulation of human mast cells by flavonoids. *Biol Pharm Bull,* December 2003, vol. 26 ((12)), 1685-90
- **ZHANG J ; BERENSTEIN E ; SIRAGANIAN RP.** Phosphorylation of Tyr342 in the linker region of Syk is critical for Fc epsilon RI signalling in mast cells. *Mol Cell Biol,* December 2002, vol. 22 ((23)), 8144-54
- **HUCKLE WR ; DY RC ; EARP HS.** Calcium-dependent increase in tyrosine kinase activity stimulated by angiotensin II. *Proc Natl Acad Sci U S A,* 15 September 1992, vol. 89 (18), 8837-41
- **ISAKOV N ; WANGE RL ; WATTS JD ; AEBERSOLD R ; SAMELSON LE.** Purification and characterization of human ZAP-70 protein-tyrosine kinase from a baculovirus expression system. *J Biol Chem,* 28 June 1996, vol. 271 (26), 15753-61

- **NICHOLSON CD ; CHALLISS RA ; SHAHID M.** Differential modulation of tissue function and therapeutic potential of selective inhibitors of cyclic nucleotide phosphodiesterase isoenzymes. *Trends Pharmacol Sci,* January 1991, vol. 12 ((1)), 19-27
- **CORTIJO J ; BOU J ; BELETA J ; CARDELUS I ; LLENAS J ; MORCILLO E et al.** Investigation into the role of phosphodiesterase IV in bronchorelaxation, including studies with human bronchus. *Br J Pharmacol,* February 1993, vol. 108 ((2)), 562-8
- **MAGNUSSON ; KLIGMAN.** *J. Invest. Dermatol.,* 1969, vol. 52, 268-276